(19) Europäisches Patentamt
European Patent Office
Office européen des brevets

(11) **EP 2 488 161 B1**

(12) **FASCICULE DE BREVET EUROPEEN**

(45) Date de publication et mention de la délivrance du brevet:
**20.04.2016 Bulletin 2016/16**

(21) Numéro de dépôt: **10773994.8**

(22) Date de dépôt: **15.10.2010**

(51) Int Cl.:
*A61K 9/16* *(2006.01)*    *A61K 9/20* *(2006.01)*
*A61K 9/48* *(2006.01)*    *A61K 9/50* *(2006.01)*
*A61K 31/485* *(2006.01)*

(86) Numéro de dépôt international:
**PCT/IB2010/054674**

(87) Numéro de publication internationale:
**WO 2011/045769 (21.04.2011 Gazette 2011/16)**

(54) **FORME PHARMACEUTIQUE ORALE SOLIDE ANTI-MÉSUSAGE ET DOTÉE D'UN PROFIL SPÉCIFIQUE DE LIBÉRATION MODIFIÉE**

MISSBRAUCHSSICHERE, ORALE FESTE DOSIERUNGSFORM MIT EINEM MODIFIZIERTEN SPEZIFISCHEN FREISETZUNGSPROFIL

ANTI-MISUSE SOLID ORAL DOSAGE FORM PROVIDED HAVING A MODIFIED SPECIFIC RELEASE PROFILE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **16.10.2009 FR 0957270**
**16.10.2009 US 252317 P**

(43) Date de publication de la demande:
**22.08.2012 Bulletin 2012/34**

(73) Titulaire: **Flamel Ireland Limited Dublin 2 (IE)**

(72) Inventeurs:
• **CASTAN, Catherine**
**F-69530 Orlienas (FR)**

• **DAVIAUD-VENET, Anne-Sophie**
**F-69230 Saint-Genis-Laval (FR)**

(74) Mandataire: **Nony**
**3, rue de Penthièvre**
**75008 Paris (FR)**

(56) Documents cités:
**WO-A1-2009/036812    WO-A2-2004/026256**
**WO-A2-2008/027993**

Remarques:
Le dossier contient des informations techniques présentées postérieurement au dépôt de la demande et ne figurant pas dans le présent fascicule.

EP 2 488 161 B1

**Description**

La présente invention vise à proposer une forme pharmaceutique solide orale, renfermant au moins un agent viscosifiant et un actif formulé à l'état de microparticules, ces dernières étant résistantes au broyage, de façon à éviter le mésusage et aptes à procurer un profil de libération modifiée spécifique comportant plusieurs phases de libération dont une au moins dépendante du pH.

D'une manière générale, les formes pharmaceutiques orales solides classiques, gélules ou comprimés, offrent une résistance insuffisante à l'extraction du principe actif qu'elles contiennent, et à ce titre peuvent faire l'objet d'un mésusage.

Ainsi, il existe un certain nombre de médicaments qui font l'objet d'un abus c'est-à-dire que leur utilisation est détournée de l'indication pour laquelle ils ont été autorisés, pour au contraire être utilisés à des fins d'obtenir un effet euphorisant, comparable à celui procuré par les drogues illicites. A titre illustratif de ces médicaments, peuvent notamment être cités les narcotiques.

Les formes galéniques solides médicamenteuses plus particulièrement concernées par ce mésusage sont les formes à libération prolongée à travers lesquelles les abuseurs peuvent disposer d'une dose plus élevée d'actif par comprimé ou gélule. Il suffit en général de broyer la gélule ou le comprimé pour « casser » l'effet de prolongation de la libération et transformer le produit à libération prolongée en produit à libération immédiate. Par la suite, la poudre obtenue peut être soit inhalée ou avalée, soit encore soumise à d'autres procédés d'extraction liquide pour préparer un liquide injectable (extraction dans des petits volumes de solvants propres à l'injection intra veineuse) ou buvable (poudre mélangée à une boisson alcoolisée ou non).

Pour prévenir ce type d'agissement, il apparaît indispensable de pouvoir disposer de formes pharmaceutiques solides orales, non compatibles avec tout autre usage que l'usage ou les usages thérapeutiques officiellement approuvés par les autorités de santé publique compétentes.

Des moyens anti-mésusage ont déjà été proposés pour des formes pharmaceutiques. Par exemple, le document WO 2007/054378 propose des formes pharmaceutiques orales et solides, constituées de microparticules de principe actif à libération prolongée, résistantes au broyage. Ces microparticules peuvent être en outre associées à un agent viscosifiant et/ou un agent séquestrant.

Toutefois, les microparticules décrites dans ce document possèdent un enrobage insensible au pH, et à ce titre, ne peuvent libérer le principe actif qu'elles contiennent, que selon un mode prolongé, continu et régulier. En conséquence, les formes pharmaceutiques décrites dans WO 2007/054378 ne s'avèrent pas aptes à procurer un profil de libération modifiée spécifique comportant plusieurs phases de libération dont une au moins dépendante du pH.

Or, certains principes actifs ou objectifs thérapeutiques requièrent des profils de libération très différents, comportant différentes phases de libération dont certaines dépendantes du pH. Cela permet notamment de faire coïncider la libération du principe actif avec des zones ciblées du petit intestin et ainsi optimiser le profil d'action du principe actif.

La présente invention vise précisément à pallier ce défaut, en proposant des formes pharmaceutiques orales solides, dotées de propriétés anti-mésusages, et qui parallèlement, sont aptes à procurer un profil de libération modifiée comportant plusieurs phases de libération dont une au moins dépendante du pH.

Plus précisément, la présente invention concerne une forme pharmaceutique solide orale, à libération modifiée d'au moins un principe actif, contenant au moins un agent viscosifiant et des microparticules contenant ledit actif, et caractérisée en ce que lesdites microparticules possèdent un diamètre moyen allant de 100 à 600 $\mu$m, et sont formées d'un coeur contenant au moins ledit principe actif et enrobé d'au moins une couche d'enrobage,

- ledit coeur étant formé d'une particule support recouverte d'une couche comprenant au moins ledit actif, et
- ladite couche d'enrobage étant formée d'un matériau composé d'au moins :

    - 25 à 70 % en poids par rapport au poids total dudit enrobage d'au moins un polymère A insoluble dans l'eau,
    - 30 à 75 % en poids par rapport au poids total dudit enrobage d'au moins un polymère B insoluble dans l'eau en dessous de pH 5 et soluble dans l'eau au dessus de pH 7, et
    - 0 à 25 % en poids par rapport au poids total dudit enrobage d'au moins un plastifiant,

- ladite couche d'enrobage représentant au moins 35 % en poids, par rapport au poids total de ladite microparticule.

En particulier, l'enrobage des microparticules selon l'invention comprend les deux polymères A et B dans un rapport pondéral polymère(s) B/polymère(s) A compris entre 0,75 et 4.

Comme il ressort de ce qui précède, une forme solide orale selon l'invention comprend au moins un agent viscosifiant sous une forme isolée desdites microparticules d'actif. Autrement dit, il n'est pas présent dans le coeur des microparticules d'actif, ni au niveau de leur couche d'enrobage.

Ledit agent viscosifiant et lesdites microparticules d'actif constituent ainsi deux entités distinctes, toutes les deux contenues dans la forme pharmaceutique solide orale de l'invention.

De préférence, la forme solide orale selon l'invention ne comprend d'agent(s) viscosifiant(s) que sous une forme isolée desdites microparticules d'actif.

On désigne au sens de l'invention de façon générale par « forme pharmaceutique solide orale », aussi bien des comprimés, des poudres, des gélules ou autres analogues, destinés à l'administration par voie orale chez l'homme ou pour usage vétérinaire.

Au regard de leurs spécificités, les formes solides orales selon l'invention sont avantageusement dotées de propriétés anti-mésusage efficaces.

On désigne au sens de l'invention de façon générale par « forme dotée de propriétés anti-mésusage», une forme pharmaceutique dont les propriétés physico-chimiques sont telles que l'usage du médicament à d'autres fins que celles autorisées, est rendu très difficile.

Plus particulièrement, comme il ressort des exemples présentés ci-après, les microparticules de la forme solide orale selon l'invention s'avèrent particulièrement résistantes au broyage, de sorte qu'il est très difficile de rompre leur enrobage et d'accéder par ce moyen au principe actif sous une forme immédiatement absorbable.

Par l'expression « résistantes au broyage », on entend signifier que les microparticules selon l'invention sont telles qu'elles permettent, en cas de broyage, le maintien du profil spécifique de libération modifiée pour au moins 40 %, de préférence au moins 60 %, et plus préférentiellement encore au moins 80 % des microparticules à libération modifiée d'actif.

Le broyage ici envisagé peut être par exemple tout broyage effectué selon les techniques habituellement mises en oeuvre par les auteurs de mésusage, comme par exemple : mortier/pilon, moulin à café, écrasement entre deux cuillères, en croquant/mastiquant, etc. Un test de broyage utilisable pour mesurer la résistance au broyage et reposant sur la technique du mortier/pilon est notamment décrit ci-dessous et mis en oeuvre dans les exemples.

Plus particulièrement, la résistance au broyage des microparticules peut être évaluée selon le protocole suivant.

Une dose de principe actif sous la forme de microparticules ou d'une forme pharmaceutique orale intacte (un comprimé ou le contenu d'une gélule) est introduite dans un mortier en pyrex de 250 ml, et broyée à l'aide du pilon en pyrex correspondant au mortier pendant 50 tours, soit pendant environ 1 minute.

Pour ce qui est du profil de libération, celui-ci peut être caractérisé par un test de dissolution. Ce test est réalisé selon la méthode de la Pharmacopée Européenne, 6ème Edition, 6.5. Chapitre 2.9.3. - Essai de dissolution des formes solides. A titre comparatif, des profils de dissolution des microparticules intactes et broyées ou de la forme pharmaceutique orale intacte et broyée peuvent être réalisés. Ces profils peuvent notamment être comparés au terme de 30 minutes de mise en contact de la poudre broyée et de la formulation intacte avec le milieu de dissolution considéré.

Une forme solide selon l'invention, n'est pas transformable ni en une forme sèche administrable par aspiration nasale et à libération immédiate de l'actif, ni en une forme injectable et à libération immédiate de l'actif, et n'est pas propice à l'extraction de l'actif par mastication et/ou broyage.

De même, lorsque la forme solide orale intacte ou broyée est introduite dans un petit volume de solvant injectable, l'agent viscosifiant présent dans la forme solide orale de l'invention va transformer le mélange en pâte inhomogène trop visqueuse pour être filtrée ou transférée dans une seringue, prévenant ainsi l'obtention d'un liquide injectable contenant le principe actif sous forme immédiatement disponible.

On entend qualifier au sens de l'invention par l'expression « à libération modifiée », l'aptitude des microparticules considérées selon l'invention à manifester au moins *in vitro*, un profil de libération de l'actif associé qui comporte trois phases, et dont les différentes séquences sont déclenchées selon deux mécanismes distincts et indépendants l'un de l'autre, l'un étant activé par le temps (1er mécanisme) et l'autre par le pH (2nd mécanisme).

Comme explicité ci-après, cette aptitude des microparticules selon l'invention est conditionnée par les spécificités de leur enrobage.

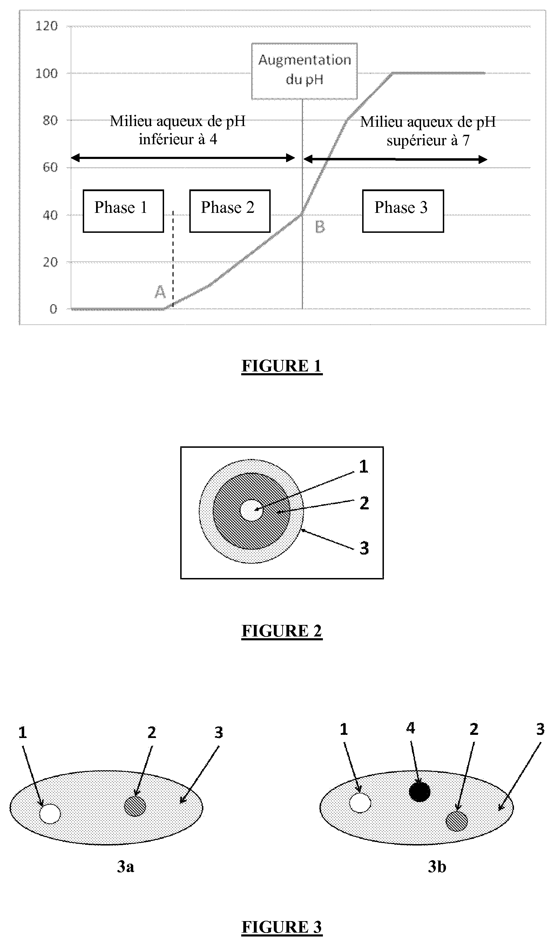

La figure 1 est une représentation schématique du profil de dissolution attendu pour une forme solide selon l'invention lorsque celle-ci est exposée successivement à

- un milieu aqueux acide de pH inférieur à 4, représentatif des conditions de pH rencontrées dans l'estomac, et
- un milieu aqueux de pH supérieur à 7, représentatif des conditions de pH rencontrées dans le petit intestin.

Dans le milieu aqueux acide de pH inférieur à 4, c'est le 1er mécanisme qui intervient. Il est actionné par le temps. Selon ce 1er mécanisme, la libération de l'actif est déclenchée (phase 2) après un temps de contact déterminé de la forme solide avec ce milieu aqueux (phase 1). Le profil de libération retardée et prolongée peut être observé avec un temps de latence donné et inférieur à 12 heures, en particulier entre 0,5 et 8 heures, voire entre 1 et 5 heures. Le temps de latence correspond au temps en dessous duquel les microparticules libèrent moins de 10 % de leur dose en actif(s).

En revanche, lorsque ces mêmes microparticules ayant séjourné dans le milieu aqueux acide de pH inférieur à 4, entrent en contact avec le milieu aqueux de pH supérieur à 7, c'est le 2nd mécanisme qui intervient. Il est actionné par le pH. Dans ce cas, la libération de l'actif est accélérée dès la mise en contact (phase 3) des microparticules avec ce milieu aqueux.

Ces deux mécanismes de libération de l'actif ou des actifs formulés dans la forme solide selon l'invention sont donc de manière générale assurés en séquence. Autrement dit, le passage de la première phase à la deuxième phase est déclenché *in vivo* par un temps de contact avec le milieu acide du milieu stomacal, alors que le passage de la deuxième phase à la troisième phase est déclenché par le changement de pH rencontré lorsque les microparticules quittent l'estomac pour entrer dans l'intestin.

Il est entendu que ces trois phases se réalisent selon l'ordre séquentiel précité lorsque l'augmentation de pH intervient après initiation de la phase 2. Dans l'hypothèse où l'augmentation de pH, déclenchant le 2nd mécanisme, interviendrait au cours de la phase 1 ou à son terme, la phase 3 interviendrait alors précocement.

Il est à noter que ce profil de libération modifiée se distingue des profils de libération obtenus avec les enrobages entériques, lesquels n'ont qu'un seul mécanisme de libération déclenché par le pH, avec pour résultat une libération négligeable tant que la forme séjourne en milieu acide. Ainsi, les enrobages entériques ne permettent pas la libération de l'actif dans l'estomac.

Selon un mode de réalisation particulier, la forme solide orale selon l'invention peut comprendre en outre au moins un agent séquestrant tel que décrit plus précisément par la suite.

Selon un autre mode de réalisation particulier, la forme solide orale selon l'invention peut comporter en outre un ou plusieurs excipients distincts des microparticules à libération modifiée.

La présente invention s'avère plus particulièrement avantageuse au regard d'actifs, appelés indifféremment « principes actifs », notamment pharmaceutiques ou vétérinaires, dont l'abus peut donner lieu à des conduites toxicomaniaques, comme par exemple ceux classés dans la catégorie des produits stupéfiants, des analgésiques ou des narcotiques. Pour des raisons évidentes, elle n'est toutefois pas limitée à la mise en oeuvre de ce type d'actif.

## Système microparticulaire

La forme pharmaceutique solide orale selon l'invention comprend des microparticules à libération modifiée dont la composition et l'architecture sont ajustées, d'une part, pour les rendre résistantes au broyage et d'autre part, pour conférer le profil spécifique de libération modifiée recherché pour l'actif ou mélange d'actifs qu'elles contiennent.

Comme précisé précédemment, les microparticules considérées selon l'invention possèdent un diamètre moyen allant de 100 à 600 $\mu$m.

De préférence, les microparticules possèdent un diamètre moyen allant de 150 à 350 $\mu$m, plus particulièrement de 200 à 300 $\mu$m, en particulier de 250 à 300 $\mu$m.

Le diamètre moyen est déterminé par diffraction laser.

D'une manière générale, l'usage de la méthode par diffraction laser, notamment telle qu'exposée dans Pharmacopée 6ème Edition, chapitre 2.9.31., pour caractériser une taille, en diamètre moyen en volume, est privilégié jusqu'à une échelle de taille de 700 $\mu$m.

Plus particulièrement, le diamètre moyen équivalent en volume des microparticules de l'invention, noté D(4 ;3), peut être obtenu selon le protocole de mesure suivant.

La distribution de tailles des particules est mesurée par diffraction laser à l'aide d'un appareil Mastersizer® 2000 de Malvern Instrument équipé d'un échantillonneur de poudre sèche de type Sirocco 2000. A partir de la répartition granulométrique mesurée sur une large gamme, le diamètre moyen équivalent en volume ou D(4;3) est calculé selon la formule suivante :

$D(4;3) = \Sigma(d^4) / \Sigma(d^3)$Les microparticules considérées selon l'invention sont structurellement organisées en un coeur enrobé ou pelliculé par un enrobage. Cette structure est représentée figure 2.

## Coeur des microparticules

Le coeur des microparticules selon l'invention est avantageusement de forme compacte et globalement sphérique.

Le coeur des microparticules selon l'invention est plus particulièrement un granulé obtenu par l'application d'une couche formée en tout ou partie de l'actif sur une particule support.

Ainsi, les microparticules selon l'invention, comme représenté schématiquement en figure 2, comportent chacune une particule support, au moins une couche active comprenant le (ou les) actif(s) et recouvrant la particule support, et au moins un enrobage permettant la libération modifiée de l'actif.

Les particules support peuvent être :

- des cristaux ou des sphères de lactose, de saccharose (comme par exemple Compressuc® PS de Tereos), de cellulose microcristalline (comme par exemple Avicel® de FMC Biopolymer, Cellet® de Pharmatrans ou Celphere® d'Asahi Kasei), de chlorure de sodium, de carbonate de calcium (comme par exemple Omyapure® 35 de Omya), d'hydrogénocarbonate de sodium, de phosphate dicalcique (comme par exemple Dicafos® AC 92-12 de Budenheim) ou de phosphate tricalcique (comme par exemple Tricafos® SC93-15 de Budenheim) ;

- des sphères ou granulés composites, par exemple les sphères de sucre fabriquées par granulation du saccharose avec de l'amidon utilisé comme liant (comme par exemple Suglets® de NP Pharm), les sphères de carbonate de calcium fabriquées avec comme liant de l'amidon (comme par exemple Destab® 90 S Ultra 250 de Particle Dynamics) ou de la maltodextrine (Hubercal® CCG4100 d'Huber).

Les particules support peuvent également être toutes autres particules d'excipient(s) pharmaceutiquement acceptable(s) comme par exemple des particules d'hydroxypropyl cellulose (comme par exemple Klucel® d'Aqualon), des particules de gomme guar (comme par exemple Grinsted® Guar de Danisco), des particules de xanthane (comme par exemple Xantural® 180 de CPKelco).

Avantageusement, la particule support a un diamètre moyen inférieur ou égal à 300 $\mu$m, de préférence compris entre 50 et 250 $\mu$m, en particulier entre 70 et 150 $\mu$m.

Selon un mode de réalisation particulier de l'invention, les particules support sont des sphères de sucre ou des sphères de cellulose microcristalline, comme par exemple les Cellet® 90 commercialisées par Pharmatrans et dont le diamètre moyen en volume est égal à environ 95 $\mu$m, ou encore les Celphere® SCP 100 et plus particulièrement la fraction de Celphere® SCP 100 inférieure à 100 $\mu$m après tamisage sur un tamis de 100 $\mu$m et dont le diamètre moyen en volume est d'environ 100 $\mu$m, ou encore des particules de phosphate dicalcique, par exemple le Dicafos® AC 92-12 et plus particulièrement la fraction de Dicafos® AC 92-12 comprise entre 50 et 100 $\mu$m après tamisage du Dicafos® AC 92-12 sur des tamis de 50 $\mu$m et 100 $\mu$m et dont le diamètre moyen en volume est d'environ 75 $\mu$m.

La couche active recouvrant la particule support pour former le coeur des microparticules de l'invention peut éventuellement comporter, outre le ou les actif(s), un ou des agent(s) liant(s) sélectionné(s) parmi :

- l'hydroxypropylcellulose (comme par exemple Klucel® EF d'Aqualon-Hercules), l'hydroxy-propylmethylcellulose (ou hypromellose) (comme par exemple Methocel® E3 ou E5 de Dow), la methylcellulose (comme par exemple Methocel® A15 de Dow),
- la polyvinylpyrrolidone (ou povidone) (comme par exemple Plasdone® K29/32 d'ISP ou Kollidon® 30 de BASF), le copolymère de vinylpyrrolidone et d'acétate de vinyl (ou copovidone) (comme par exemple Plasdone® S630 d'ISP ou Kollidon® VA 64 de BASF),
- le dextrose, les amidons prégélatinés, la maltodextrine.

Les agents liants préférés sont la povidone (Plasdone® K29/32 d'ISP), l'hydroxypropylcellulose (Klucel® EF d'Aqualon-Hercules) ou l'hypromellose (Methocel® E3 ou E5 de Dow).

La couche contenant au moins un actif et recouvrant la particule support représente au moins 50 % en poids, de préférence au moins 60 % en poids, de préférence encore de 70 à 95 % en poids et en particulier de 80 à 90 % en poids du poids du granulé.

La couche active recouvrant la particule support pour former le coeur des microparticules de l'invention peut également comporter optionnellement, outre le (ou les) actif(s), un ou plusieurs excipients physiologiquement acceptables, tels que des tensioactifs, des désintégrants, des charges, des agents contrôlant ou modifiant le pH (tampons), des agents anti-mousse dont le choix et l'ajustement des quantités relèvent clairement des compétences de l'homme de l'art.

Actifs

En ce qui concerne l'actif, pour des raisons évidentes, il est manifeste que les microparticules considérées selon l'invention sont compatibles avec une grande diversité d'actifs.

Toutefois, les formes solides selon l'invention sont particulièrement avantageuses au regard de la mise en oeuvre d'actifs dont l'abus peut donner lieu à des conduites toxicomaniaques, comme par exemple des actifs classés dans la catégorie des produits stupéfiants, des analgésiques ou des narcotiques.

Ainsi l'actif contenu dans les microparticules enrobées selon l'invention peut être, par exemple, avantageusement choisi parmi au moins l'une des familles de substances actives suivantes : les amphétamines, les analgésiques, les anorexigènes, les antidépresseurs, les antiépileptiques, les antiparkinsoniens, les anxiolytiques, les barbituriques, les benzodiazépines, les hypnotiques, les narcotiques, les neuroleptiques, les psychostimulants et les psychotropes.

Dans le cas où l'actif est un narcotique, il s'agit, de préférence, d'un opioïde.

Plus précisément, le narcotique mis en oeuvre peut être choisi parmi l'oxycodone, l'oxymorphone, l'hydromorphone, l'hydrocodone, le tramadol et leurs sels pharmaceutiquement acceptables.

**Enrobage des microparticules**

Dans le cadre de la présente invention, le coeur, contenant l'actif ou un mélange d'actifs, est recouvert d'un enrobage dont la composition et l'épaisseur sont précisément ajustées pour d'une part procurer le profil de libération spécifique de l'actif *in vitro* en trois phases déclenchées par deux mécanismes de libération indépendant, l'un activé par le temps et l'autre par le pH et d'autre part contribuer à rendre ces microparticules à libération modifiée résistantes au broyage.

L'enrobage qui recouvre le coeur des microparticules représente au moins 35 % en poids du poids total de la microparticule à libération modifiée, soit un taux d'enrobage d'au moins 35 %.

Plus particulièrement, l'enrobage peut représenter de 35 à 60 % en poids, en particulier de 40 à 55 % en poids, plus particulièrement de 45 à 55 % en poids du poids total de la microparticule à libération modifiée.

L'enrobage peut être formé d'un matériau composite obtenu par mélange de :

- au moins un polymère A insoluble dans l'eau ;
- au moins un second polymère B insoluble dans l'eau à pH inférieur à 5 et soluble dans l'eau à pH supérieur à 7 ;
- et éventuellement au moins un agent plastifiant.

Le polymère A, insoluble dans l'eau, est plus particulièrement sélectionné parmi l'éthylcellulose, par exemple commercialisée sous la dénomination Ethocel[e], l'acétate butyrate de cellulose, l'acétate de cellulose, les copolymères d'ammonio (méth)acrylate (copolymère d'acrylate d'éthyle, de méthacrylate de méthyle et de méthacrylate de triméthylammonio éthyle) notamment ceux commercialisés sous les dénominations Eudragit® RL et Eudragit® RS, les esters d'acides poly(méth)acryliques, notamment ceux commercialisés sous la dénomination Eudragit® NE et leurs mélanges.

L'enrobage des microparticules peut contenir de 25 % à 70 % en poids de polymère(s) A par rapport à son poids total.

Selon un mode de réalisation préféré, l'enrobage des microparticules présente une teneur en polymère(s) A comprise entre 30 et 65 %, notamment entre 35 et 60 % en poids, plus particulièrement entre 35 et 55 % en poids, et encore plus particulièrement entre 35 et 50 % en poids, par rapport à son poids total.

A titre illustratif et non limitatif des polymères B convenant à l'invention, c'est à dire insolubles dans l'eau à pH inférieur à 5 et solubles dans l'eau à pH supérieur à 7, on peut notamment citer :

- le(s) copolymère(s) d'acide méthacrylique et de méthacrylate de méthyle,
- le(s) copolymère(s) d'acide méthacrylique et d'acrylate d'éthyle,
- l'acétate phtalate de cellulose (CAP),
- l'acétate succinate de cellulose (CAS),
- l'acétate trimellitate de cellulose (CAT),
- le phtalate d'hydroxypropylméthylcellulose (ou hypromellose phtalate) (HPMCP),
- l'acétate succinate d'hydroxypropylméthylcellulose (ou hypromellose acetate succinate) (HPMCAS),
- la carboxyméthyléthylcellulose,
- la gomme shellac,
- l'acétate phtalate de polyvinyle (PVAP),
- et leurs mélanges.

Selon un mode préféré de l'invention, ce polymère B est choisi parmi le(s) copolymère(s) d'acide méthacrylique et de méthacrylate de méthyle, le(s) copolymère(s) d'acide méthacrylique et d'acrylate d'éthyle et leurs mélanges.

Les polymères B se dissolvent dans l'eau à une valeur de pH donnée, comprise entre 5 et 7, cette valeur variant en fonction de leurs caractéristiques physico-chimiques intrinsèques, telles que leur nature chimique et leur longueur de chaîne.

Par exemple, le polymère B peut être un polymère dont la valeur de pH de solubilisation est de :

- 5,0 à l'image par exemple du phtalate d'hydroxypropylméthylcellulose et notamment celui commercialisé sous la dénomination HP-50 par Shin-Etsu,
- 5,5 à l'image par exemple du phtalate d'hydroxypropylméthylcellulose et notamment celui commercialisé sous la dénomination HP-55 par Shin-Etsu ou du copolymère d'acide méthacrylique et d'acrylate d'éthyle 1 :1 et notamment celui commercialisé sous la dénomination Eudragit® L100-55 de Evonik,
- 6,0 à l'image par exemple d'un copolymère d'acide méthacrylique et de méthacrylate de méthyle 1 :1 et notamment celui commercialisé sous la dénomination Eudragit® L100 de Evonik,
- 7,0 comme par exemple un copolymère d'acide méthacrylique et de méthacrylate de méthyle 1 :2 et notamment celui commercialisé sous la dénomination Eudragit S100 de Evonik.

L'ensemble de ces polymères est soluble à une valeur de pH supérieure à son pH de solubilisation.

L'enrobage est avantageusement composé d'au moins 30 à 75 %, en particulier de 30 à 70 %, en particulier de 35 à 65 %, voire de 35 à 60 % en poids de polymère(s) B par rapport à son poids total.

L'enrobage des microparticules selon l'invention comprend les deux polymères A et B dans un rapport pondéral polymère(s) B/polymère(s) A supérieur ou égal à 0,75.

Selon une autre variante de réalisation, le rapport polymère(s) B/polymère(s) A est en outre inférieur à 4, voire inférieur à 2 et plus particulièrement inférieur à 1,5.

Le rapport pondéral polymère(s) B/polymère(s) A est compris entre 0,75 et 4, et plus particulièrement entre 0,75 et 1,5.

L'enrobage des microparticules selon l'invention comprend les deux polymères A et B dans un rapport pondéral polymère(s) B/polymère(s) A compris entre 0,75 et 4.

Selon un mode de réalisation particulier, l'enrobage des microparticules est formé d'au moins un mélange comprenant, à titre de polymère A, au moins l'éthylcellulose ou l'acétate butyrate de cellulose ou le copolymère d'ammonio (méth)acrylate ou un de leurs mélanges, avec, à titre de polymère B, au moins un copolymère d'acide méthacrylique et d'acrylate d'éthyle ou un copolymère d'acide méthacrylique et de méthacrylate de méthyle ou un de leurs mélanges.

Ainsi, selon un mode de réalisation particulier, l'enrobage des microparticules selon l'invention peut être avantageusement formé d'au moins un couple polymère B/polymère A choisi parmi les couples suivants :

1. copolymère d'acide méthacrylique et d'acrylate d'éthyle, 1:1 / éthylcellulose,
2. copolymère d'acide méthacrylique et de méthacrylate de méthyle 1:2 / éthylcellulose,
3. mélange de copolymère d'acide méthacrylique et d'acrylate d'éthyle 1:1 et de copolymère d'acide méthacrylique et de méthacrylate de méthyle, 1:2 / éthylcellulose,
4. copolymère d'acide méthacrylique et d'acrylate d'éthyle 1:1 / acétate butyrate de cellulose,
5. copolymère d'acide méthacrylique et de méthacrylate de méthyle 1:2 / acétate butyrate de cellulose,
6. mélange de copolymère d'acide méthacrylique et d'acrylate d'éthyle 1:1 et de copolymère d'acide méthacrylique et de méthacrylate de méthyle 1:2 / acétate butyrate de cellulose,
7. copolymère d'acide méthacrylique et d'acrylate d'éthyle 1:1 / acétate de cellulose,
8. copolymère d'acide méthacrylique et de méthacrylate de méthyle 1:2 / acétate de cellulose,
9. mélange de copolymère d'acide méthacrylique et d'acrylate d'éthyle 1:1 et de copolymère d'acide méthacrylique et de méthacrylate de méthyle 1:2 / acétate de cellulose,
10. copolymère d'acide méthacrylique et d'acrylate d'éthyle 1:1 / copolymère d'ammonio (méth)acrylate,
11. copolymère d'acide méthacrylique et de méthacrylate de méthyle 1:2 / copolymère d'ammonio (méth)acrylate, et
12. mélange de copolymère d'acide méthacrylique et d'acrylate d'éthyle 1:1 et de copolymère d'acide méthacrylique et de méthacrylate de méthyle 1:2 / copolymère d'ammonio (méth)acrylate.

Selon un mode de réalisation particulièrement préféré, l'enrobage comprend au moins le couple polymère B/polymère A, le polymère B étant formé du mélange de copolymère d'acide méthacrylique et d'acrylate d'éthyle 1:1 et de copolymère d'acide méthacrylique et de méthacrylate de méthyle 1:2, et le polymère A étant l'éthylcellulose.

L'enrobage des microparticules selon l'invention peut comprendre en outre au moins un agent plastifiant.

Cet agent plastifiant peut être notamment choisi parmi :

- le glycérol et ses esters, et de préférence parmi les glycérides acétylés, glycéryl-mono-stéarate, glycéryl-triacétate, glycéryl-tributyrate,
- les phtalates, et de préférence parmi les dibutylphtalate, diéthylphtalate, diméthylphtalate, dioctylphtalate,
- les citrates, et de préférence parmi les acétyltributylcitrate, acétyltriéthylcitrate, tributylcitrate, triéthylcitrate,
- les sébaçates, et de préférence parmi les diéthylsébaçate, dibutylsébaçate,
- les adipates,
- les azélates,
- les benzoates,
- le chlorobutanol,
- les polyéthylènes glycols,
- les huiles végétales,
- les fumarates, de préférence le diéthylfumarate,
- les malates, de préférence le diéthylmalate,
- les oxalates, de préférence le diéthyloxalate,
- les succinates ; de préférence le dibutylsuccinate,
- les butyrates,
- les esters de l'alcool cétylique,

- les malonates, de préférence le diéthylmalonate,
- l'huile de ricin,
- et leurs mélanges.

En particulier, l'enrobage peut comprendre moins de 25 % en poids, de préférence de 5 % à 20 % en poids, et plus préférentiellement de 10 % à 20 % en poids d'agent(s) plastifiant(s), par rapport à son poids total.

Ainsi, l'enrobage de particules selon l'invention peut être avantageusement formé d'au moins:

- 30 à 60 %, en particulier de 35 à 55 % en poids d'au moins un polymère A choisi parmi l'éthylcellulose, l'acétate butyrate de cellulose, l'acétate de cellulose, un copolymère d'ammonio (méth)acrylate ou un de leurs mélanges,
- de 30 à 70 %, en particulier de 30 à 60 % en poids d'au moins un polymère B choisi parmi un copolymère d'acide méthacrylique et de méthacrylate de méthyle, notamment un copolymère d'acide méthacrylique et de méthacrylate de méthyle 1:1 ou un copolymère d'acide méthacrylique et de méthacrylate de méthyle 1:2; un copolymère d'acide méthacrylique et d'acrylate d'éthyle, notamment un copolymère d'acide méthacrylique et d'acrylate d'éthyle 1:1 ou un copolymère d'acide méthacrylique et d'acrylate d'éthyle 1:2, et leurs mélanges,
- et de 10 à 20 % en poids d'au moins un agent plastifiant tel que par exemple le triéthylcitrate ou le polyéthylène glycol.

A titre illustratif et non limitatif des particules conformes à l'invention, on peut notamment citer celles dont l'enrobage possède l'une des compositions suivantes :

- 35 à 55 % d'éthylcellulose

  30 à 60 % d'un mélange de copolymère d'acide méthacrylique et d'acrylate d'éthyle 1 :1 et de copolymère d'acide méthacrylique et de méthacrylate de méthyle 1:2
  10 à 20 % de triéthylcitrate

- 35 à 55 % d'acétate butyrate de cellulose

  30 à 60 % de copolymère d'acide méthacrylique et d'acrylate d'éthyle 1 :1
  10 à 20 % de triéthylcitrate

- 35 à 55 % d'éthylcellulose

  30 à 60 % d'un mélange de copolymère d'acide méthacrylique et d'acrylate d'éthyle 1 :1 et de copolymère d'acide méthacrylique et de méthacrylate de méthyle 1:2
  10 à 20 % de polyéthylène glycol

Bien entendu, l'enrobage peut comprendre divers autres adjuvants additionnels utilisés classiquement dans le domaine de l'enrobage. Il peut s'agir, par exemple :

- de pigments et de colorants, comme le dioxyde de titane, le sulfate de calcium, le carbonate de calcium précipité, les oxydes de fer, les colorants alimentaires naturels tels que les caramels, les caroténoïdes, le carmin, les chlorophyllines, Rocou (ou annatto), les xanthophylles, les anthocyanes, la bétanine, l'aluminium et les colorants de synthèse alimentaires comme les jaunes n° 5 et n° 6, les rouges n° 3 et n°40, le vert n° 3 et le vert Emeraude, les bleus n° 1 et n° 2 ;
- de charges, comme le talc, le stéarate de magnésium, le silicate de magnésium :
- d'agents anti-mousse, comme la simethicone, la dimethicone ;
- d'agents tensioactifs, comme les phospholipides, les polysorbates, les stéarates de polyoxyéthylène, les esters d'acide gras et de sorbitol polyoxyéthyléné, les huiles de ricin hydrogénées polyoxyéthylénées, les éthers d'alkyle polyoxyéthylénés, le monooléate de glycérol,
- et leurs mélanges.

Selon un mode de réalisation particulier de l'invention, l'enrobage des microparticules selon l'invention ne contient pas de principe actif.

Selon un autre mode de réalisation de l'invention, l'enrobage est dénué de composé soluble à une valeur de pH allant de 1 à 4.

L'enrobage peut être mono- ou multi-couche. Selon une variante de réalisation, il est composé d'une unique couche formée à partir du matériau composite défini précédemment.

Selon un mode de réalisation particulièrement préféré de l'invention, l'enrobage comprend moins de 30 % en poids, par rapport à son poids total, d'agent(s) lubrifiant(s), en particulier moins de 20 %, notamment moins de 10 %, plus particulièrement moins de 5 % en poids, voire est avantageusement totalement dépourvu d'agent lubrifiant.

On entend désigner par « agent lubrifiant », encore appelé « agent de glissement », une substance servant à réduire l'agglomération des polymères pendant la phase d'enrobage des microparticules.

En particulier, l'enrobage des microparticules selon l'invention comprend à ce titre moins de 30 % en poids de talc, par rapport à son poids total, de préférence moins de 20 %, notamment moins de 10 %, de préférence moins de 5 % en poids, voire est avantageusement dépourvu de talc.

## Agent viscosifiant

Comme précisé précédemment, une forme solide orale selon l'invention comprend en outre au moins un agent viscosifiant destiné à renforcer la prévention à l'égard d'un mésusage intentionnel de l'actif contenu dans la forme solide orale.

Plus précisément il a pour finalité, lorsque la forme solide orale est mise au contact d'un petit volume de solvant injectable, de transformer le mélange correspondant en une pâte inhomogène, trop visqueuse pour être filtrée ou transférée dans une seringue, prévenant ainsi l'obtention d'un liquide injectable contenant le principe actif sous forme immédiatement disponible.

Au sens de l'invention, une forme solide orale selon l'invention comprend donc au moins un agent viscosifiant sous une forme isolée des microparticules d'actif

De préférence, la forme solide orale selon l'invention ne comprend d'agent(s) viscosifiant(s) que sous une forme isolée des microparticules d'actif.

Selon un mode de réalisation préféré, l'agent viscosifiant est choisi parmi les agents viscosifiants solubles dans au moins l'un des solvants choisi parmi l'eau, les alcools, les cétones et leurs mélanges.

Selon un mode de réalisation préféré, l'agent viscosifiant est apte à augmenter la viscosité d'un petit volume (entre 2,5 mL et 10 mL) de solvant, de façon à empêcher l'injection par voie intra-veineuse. De fait, la viscosité devient tellement élevée que le soutirage du mélange formé par la mise en oeuvre de la forme solide orale selon l'invention dans un faible volume de solvant injectable par une seringue devient impossible.

Selon un mode de réalisation particulier, une forme solide selon l'invention peut avantageusement comprendre un mélange de plusieurs agents viscosifiants qui sera efficace à la fois dans le cas d'une extraction en phase aqueuse et dans un solvant organique.

S'agissant de la quantité d'agent viscosifiant, elle est aisément déterminable par l'homme du métier. Cette quantité correspond avantageusement à la quantité minimale nécessaire pour rendre la viscosité de 2,5 mL de liquide d'extraction à une valeur supérieure ou égale à 100 mPa.s, de préférence 200 mPa.s, et plus préférentiellement encore au-delà de 500 mPa.s, et mieux encore 1000 Pa.s.

Selon un mode de réalisation particulier, l'agent viscosifiant est choisi parmi :

- les polyacides acryliques, en particulier les carbomer, par exemple les Carbopol®,
- les polyalkylènes glycols, par exemple les polyéthylènes glycol,
- les polyoxydes d'alkylène, par exemple les polyoxydes d'éthylène ou polyoxyéthylènes,
- les polyvinylpyrrolidones,
- les gélatines,
- les polysaccharides, de préférence choisis parmi l'alginate de sodium, les pectines, la gomme guar, les xanthanes, les carraghénanes, les gellanes, l'hydroxypropylcellulose, l'hydroxypropylméthylcellulose, la méthylcellulose, l'hydroxyéthylcellulose et la carboxyméthylcellulose,
- et leurs mélanges.

Selon un mode de réalisation particulièrement préféré, l'agent viscosifiant est un polyoxyéthylène, en particulier un polyoxyéthylène possédant un haut poids moléculaire, et plus particulièrement un polyoxyéthylène ayant un poids moléculaire moyen allant de 1 million g/mole à environ 8 millions g/mole.

A titre d'agent viscosifiant, on peut citer en particulier le polyoxyéthylène commercialisé par la société Dow sous la référence Sentry Polyox WSR® 303.

L'agent viscosifiant, par exemple le polyoxéthylène à haut poids moléculaire, est sous forme de microparticules, distinctes des microparticules à libération modifiée d'actif selon l'invention telles que décrites précédemment.

Avantageusement, les microparticules d'agent viscosifiant ont une distribution de taille proche de celle des microparticules à libération modifiée d'actif selon l'invention, de sorte qu'elles ne sont pas séparables des microparticules d'actif par tamisage.

Avantageusement, le diamètre moyen en volume des microparticules d'agent viscosifiant est compris entre 0,5 et 2 fois, de préférence compris entre 0,7 et 1,5 fois, de préférence encore compris entre 0,8 et 1,25 fois le diamètre moyen en volume des microparticules à libération modifiée d'actif.

**Agent séquestrant**

Selon un autre mode de réalisation particulier, la forme solide orale selon l'invention peut comprendre en outre au moins un agent séquestrant.

L'agent séquestrant va notamment permettre de capter l'actif qui pourrait être extrait des microparticules de l'invention, après un séjour de plusieurs heures dans une boisson, et le rendre ainsi non disponible pour une absorption immédiate.

Plus particulièrement, l'agent séquestrant est un composé ionique, apte à former en solution, par exemple dans une boisson aqueuse ou alcoolisée, un complexe avec l'actif lui-même sous forme ionisée, et notamment un complexe faiblement soluble.

Ainsi, lorsque l'actif et l'agent séquestrant se trouvent simultanément dans un solvant adapté, par exemple dans le cas d'une tentative illicite d'extraction de l'actif, l'agent séquestrant est apte à induire une complexation ou une interaction chimique avec l'actif dans ledit solvant. Au sens de la présente invention, un solvant adapté est un solvant usuel choisi parmi l'eau et les solutions aqueuses, telles que les mélanges eau-éthanol, l'alcool, les boissons alcoolisées, les sodas, le vinaigre, l'eau oxygénée, et leurs mélanges.

Les agents séquestrants utilisés pour piéger l'actif sont inoffensifs y compris pour un usage régulier. Ce sont des produits inertes du point de vue pharmacologique et approuvés par les différentes pharmacopées et autorités d'enregistrement des médicaments.

Si présent, l'agent séquestrant est sous forme de microparticules distinctes des microparticules à libération modifiée d'actif.

Selon un mode de réalisation particulier, l'agent séquestrant comprend un sel, lequel contient des ions aptes à former un complexe avec l'actif en solution. Si en solution, l'actif est sous forme cationique, l'agent séquestrant est un composé anionique. De la même manière, lorsque l'actif en solution est sous forme anionique, l'agent séquestrant est un composé cationique.

De façon non limitative, parmi les agents séquestrants anioniques, on peut citer les composés suivants :

- le dodécyl sulfate de sodium, le docusate de sodium ;
- les polymères anioniques, tels que les polyacides acryliques réticulés (ou carbomer par exemple le Carbopol®), les sels de carboxyméthycellulose comme la carmellose sodique ou la carmellose calcique, la carboxyméthylcellulose réticulée comme la croscarmellose sodique et ses dérivés, les polysaccharides, par exemple les alginates, la gomme xanthane ou arabique, l'alginate-(sulfonate)propylène glycol ;
- les sels mono- ou polyvalents, tels que les glucuronates, citrates, acétates, carbonates, gluconates, succinates, phosphates, glycérophosphates, les lactates, les trisilicates, les fumarates, les adipates, les benzoates, les salicylates, les tartrates, les sulfonamides, les acésulfames ;
- les acides gras saponifiés, tels que les sels d'acide stéarique, les sels d'acide palmitique ;
- les polyamino acides, protéines ou peptides anioniques, tels que les glutamates, les aspartates, les albumines, les caséines, les globulines ;
- les résines échangeuses de cations, fortement acides, telles que les copolymères de styrène et de divinylbenzène sulfoniques, comme par exemple l'Amberlite® IRP69, l'Amberlite® 1R69F, l'Amberlite® 200 ou l'Amberlite® 200C, commercialisées par la société Rohm and Haas, ou Dowex® 88, commercialisé par Dow ;
- les résines échangeuses de cations, faiblement acides, telles que les copolymères réticulés d'acide méthacrylique et de divinylbenzène ou leurs sels, comme par exemple l'Amberlite® IRP88 et l'Amberlite® 1RP64, commercialisées Rohm and Haas ou WEX MAC-3® commercialisé par Dow ;
- et leurs mélanges.

Parmi les agents séquestrants cationiques, on peut citer :

- les sels d'ammoniums quaternaires, en particulier le bromure de triméthyl tétradécyl ammonium ou le chlorure de benzéthonium ;
- les polymères cationiques, tels que les chitosans et les copolymères d'acrylate d'éthyle, de méthacrylate de méthyle et de chlorure de méthacrylate de triméthylammonioéthyle (par exemple, Eudragit® RS, Eudragit® RL) et les copolymères d'acrylate d'éthyle, de méthacrylate de méthyle et d'acide méthacrylique (par exemple Eudragit® E) ;
- les polyamino acides, protéines ou peptides cationiques comme les polylysines, les polyarginines ;
- les résines échangeuses d'anions, basiques, telles que les polyamines phénoliques comme l'Amberlite® IRP58, commercialisée par Rohm and Haas, les copolymères de styrène et de divinylbenzène porteurs de fonctions ammoniums quaternaires, comme par exemple la Duolite® AP143 ou l'Amberlite® IRP67, commercialisées par Rohm and Haas, ou le DOWEX 22, commercialisé par Dow ;

- et leurs mélanges.

Selon un mode de réalisation particulier de l'invention, l'agent séquestrant est choisi parmi:

- le sodium dodécyl sulfate ou le docusate de sodium ;
- les sels d'ammoniums quaternaires, en particulier le bromure de triméthyl tétradécyl ammonium ou le chlorure de benzéthonium ;
- les résines fortement acides échangeuses de cations, lorsque l'actif en solution est cationique, ou les résines fortement basiques échangeuses d'anions lorsque l'actif en solution est anionique, selon la polarité de l'actif, et leurs mélanges.

Selon un mode de réalisation particulièrement préféré de l'invention, lorsque l'actif en solution est sous forme cationique, l'agent séquestrant est choisi parmi

- les résines échangeuses de cations, fortement acides, telles que les copolymères de styrène et de divinylbenzène sulfoniques, comme par exemple l'Amberlite® IRP69, l'Amberlite® 1R69F, l'Amberlite® 200 ou l'Amberlite® 200C, commercialisées par la société Rohm and Haas, ou Dowex® 88, commercialisé par Dow ; et
- les résines échangeuses de cations, faiblement acides, telles que les copolymères réticulés d'acide méthacrylique et de divinylbenzène ou leurs sels, comme par exemple l'Amberlite® IRP88 et l'Amberlite® 1RP64, commercialisées Rohm and Haas ou WEX MAC-3® commercialisé par Dow.

La quantité d'agent est adaptée par l'homme de l'art par un calcul de la quantité en charge ionique nécessaire pour piéger tout ou partie de la dose d'actif contenue dans la forme solide unitaire.

En particulier, la quantité d'agent séquestrant doit être telle qu'elle permet de complexer suffisamment d'actif de sorte que la quantité restante d'actif libre en solution est insuffisante pour atteindre l'effet désiré en cas d'utilisation illicite.

Particulièrement, la quantité d'agent séquestrant est telle qu'elle permet de complexer au moins 40 %, de préférence au moins 50 %, de préférence encore au moins 60 %, de préférence au moins 70 % de la dose d'actif contenue dans la forme solide unitaire.

De préférence, la quantité d'agent séquestrant est suffisante pour complexer tout l'actif de la dose unitaire.

Avantageusement, les microparticules d'agent séquestrant ont une distribution de taille proche de celle des microparticules d'actif, de sorte qu'elles ne sont pas séparables des microparticules d'actif par tamisage ou sédimentation.

De préférence, le diamètre moyen en volume des microparticules d'agent séquestrant est compris entre 0,5 et 2 fois, de préférence compris entre 0,7 et 1,5 fois, de préférence encore compris entre 0,8 et 1,25 fois le diamètre moyen en volume des microparticules d'actif.

Selon un mode de réalisation particulier, la forme solide selon l'invention peut ainsi comprendre, outre les microparticules d'actifs, des microparticules d'agent viscosifiant et des microparticules d'agent séquestrant.

## Préparation de la forme solide orale

### Coeur des microparticules

Les granulés formant le coeur desdites microparticules peuvent être obtenus par pulvérisation, dans un lit d'air fluidisé, de l'actif, avec éventuellement un ou plusieurs excipient(s) pharmaceutiquement acceptable(s), comme des agents liants, des charges, des tensio-actifs, des désintégrants, des agents tampons, des agents anti-mousse sur une particule support, telle que décrite précédemment.

Le(s) principe(s) actif(s) et les éventuels excipients sont mélangés en solution ou dispersés dans l'eau ou dans des solvants organiques pharmaceutiquement acceptables et à bas point d'ébullition comme l'éthanol, l'isopropanol, l'acétone et leurs mélanges.

### Microparticules

Selon un mode de réalisation préféré, l'enrobage disposé en surface des microparticules à libération modifiée est obtenu par pulvérisation en lit d'air fluidisé, en particulier équipé d'un Würster et selon une orientation du spray ascendante (bottom spray), d'une solution ou dispersion contenant au moins lesdits polymères A et B sur les granulés obtenus ci-dessus.

De manière préférentielle, les polymères A et B et, le cas échéant, le(s) plastifiant(s) sont pulvérisés à l'état de soluté c'est-à-dire sous une forme solubilisée dans un solvant. Ce solvant est généralement constitué de solvant(s) organique(s) mélangés ou non avec de l'eau. Le(s) solvant(s) organique(s) est/sont choisi(s) parmi les solvants connus de l'homme de l'art. A titre d'exemple, on peut citer l'acétone, l'isopropanol, l'éthanol et leurs mélanges.

L'enrobage ainsi formé s'avère homogène en terme de composition par opposition à un enrobage formé à partir d'une dispersion de ces mêmes polymères dans un milieu liquide, majoritairement aqueux, non solvant ou mauvais solvant desdits polymères A et B.

Selon une variante de réalisation préférée, la solution pulvérisée contient moins de 40 % en poids d'eau, en particulier moins de 30 % en poids d'eau et plus particulièrement moins de 25 % en poids d'eau, voire une teneur en eau inférieur ou égale à 10 % en poids par rapport au poids total de solvants.

*Forme pharmaceutique orale solide*

Selon un mode de réalisation particulièrement préféré, une forme pharmaceutique orale solide selon l'invention est un comprimé ou une gélule.

Dans le cas de la présentation sous la forme d'une gélule, les microparticules à libération modifiée d'actif, les microparticules d'agent viscosifiant et les éventuelles microparticules d'agent séquestrant sont mélangées au préalable avec des excipients connus de l'homme de l'art comme par exemple des diluants, des lubrifiants ou des agents d'écoulement, tels que décrits plus précisément par la suite. Le mélange obtenu est ensuite réparti dans des gélules. Alternativement, un mode de remplissage séquentiel des gélules peut être mis en oeuvre, les différents composants étant ajoutés les uns après les autres ou sous forme de mélange(s) partiel(s).

Dans le cas de la présentation sous la forme d'un comprimé, les microparticules à libération modifiée d'actif, les microparticules d'agent viscosifiant et les éventuelles microparticules d'agent séquestrant sont mélangées au préalable avec des excipients connus de l'homme de l'art comme des lubrifiants ou agents d'écoulement, des diluants ou agents de compression, tels que décrits plus précisément par la suite. Le mélange est ensuite comprimé.

La compression peut être réalisée selon toute méthode conventionnelle et sa mise en oeuvre relève clairement des compétences de l'homme de l'art.

Les comprimés possèdent avantageusement une résistance significative à la rupture. Par exemple, pour un comprimé rond de diamètre 12 mm, cette dureté peut varier de 50 à 500 N, en particulier de 60 à 200 N. Cette dureté peut être mesurée selon le protocole décrit dans la Pharmacopée Européenne 6ème édition chapitre 2.9.8.

Selon une variante de réalisation, les microparticules à libération modifiée d'actifs peuvent être mélangées avec d'autres microparticules à libération modifiée ayant des compositions d'enrobage différentes ou des tailles différentes ou encore avec des particules d'actif à libération immédiate

Il s'agit là de méthodologies générales intéressantes, qui permettent de produire les formes solides de l'invention d'une manière simple et économique.

La forme solide finale, notamment sous forme de comprimé ou gélule, peut, le cas échéant, être soumise à des traitements complémentaires, selon les techniques et formules connues de l'homme de l'art visant par exemple à former à leur surface un pelliculage ou enrobage particulier destiné à leur procurer des propriétés ou qualités complémentaires (couleur, aspect, masquage de goût, etc.).

Selon un mode de réalisation particulier, une forme solide selon l'invention, notamment de type comprimé ou gélule, présente un taux de charge en microparticules à libération modifiée d'actif, allant de 5 % à 95 % en poids par rapport à son poids total, en particulier de 10 % à 90 % en poids, et plus particulièrement de 20 à 85 % en poids.

## Excipients

Comme précisé précédemment, une forme pharmaceutique orale solide selon l'invention se présente avantageusement sous la forme d'un comprimé ou d'une gélule, contenant des microparticules d'actif telles que définies ci-dessus.

La forme pharmaceutique orale solide contenant les microparticules à libération modifiée de l'actif peut ainsi comprendre des excipients physiologiquement acceptables classiques et utiles pour formuler les microparticules au sein d'une matrice, par exemple sous forme d'un comprimé ou au sein d'un mélange inclus dans une gélule.

Selon un mode de réalisation particulier, une forme pharmaceutique orale solide selon l'invention, de type gélule, peut contenir, outre les microparticules à libération modifiée d'actif, les microparticules d'agent viscosifiant et les éventuelles microparticules d'agent séquestrant :

- des diluants, comme le lactose, le saccharose, les sphères de sucre (Suglets® de NP Pharm), la cellulose microcristalline (Avicel® de FMC Biopolymer, Cellet® de Pharmatrans, Celphere® d'Asahi Kasei), les carbonates de calcium sous forme de cristal (Omyapure® 35 de Omya) ou sous forme de particules déjà formulées avec des agents liants (le Destab® 90 S Ultra 250 de Particle Dynamics ou Hubercal® CCG4100 d'Huber), les phosphates di- et tricalciques (Dicafos® et Tricafos® de Budenheim), l'oxyde de magnésium, les phosphate et sulfate de calcium ;

- des lubrifiants ou agents d'écoulement comme les stéarates en particulier le stéarate de magnésium, le stéarate de calcium ou le stéarate de zinc, l'acide stéarique, le béhénate de glycérol, le stéaryle fumarate de sodium, le talc, la silice colloïdale ;
- des désintégrants, comme les amidons et amidons prégélatinés (amidon de maïs), la carboxymethylcellulose, la croscarmellose, la crospovidone (grades de Polyplasdone® d'ISP, le Kollidon® CL de BASF), hydroxypropylcellulose faiblement substituée ;
- des colorants ou pigments, comme le dioxide de titane, le sulfate de calcium, le carbonate de calcium précipité, les oxydes de fer, les colorants alimentaires naturels tels que les caramels, les caroténoïdes, le carmin, les chlorophyllines, le Rocou (ou annatto), les xanthophylles, les anthocyanes, la bétanine, l'aluminium, et les colorants alimentaires de synthèse comme les jaunes n° 5 et n° 6, les rouges n° 3 et n° 40, le vert n° 3 et le vert Emeraude, les bleus n° 1 et n° 2 ;
- des arômes, par exemple les arômes fraise, orange, banane, menthe ;
- des conservateurs, comme les parabens, en particulier le méthylparaben, l'éthylparaben, le propylparaben et le butylparaben, l'acide benzoïque et ses sels (par exemple le benzoate de sodium), le chlorocresol, l'acide sorbique et ses sels, la glycérine ;
- du polyéthylène glycol, de l'alcool polyvinylique, du palmitostéarate de glycérol ;

et leurs mélanges.

Le choix de ces excipients, pour une forme solide orale de type gélule, relève clairement des compétences de l'homme de l'art.

Selon un mode particulier, une forme solide selon l'invention de type gélule peut notamment comprendre un ou plusieurs diluant(s) dans une teneur allant de 0 % à 80 % en poids, en particulier de 0 % à 70 % en poids, et plus particulièrement de 35 % à 65 % en poids par rapport au poids total de la forme solide de type gélule.

En particulier, une forme solide selon l'invention, de type gélule, peut comprendre un ou plusieurs lubrifiant(s) ou agent(s) d'écoulement dans une teneur allant de 0,1 % à 5 % en poids, en particulier de 0,5 % à 2 % en poids par rapport au poids total de la forme solide de type gélule.

Selon un mode de réalisation particulier, une forme solide selon l'invention de type gélule comprend, outre les microparticules à libération modifiée d'actif, définies ci-dessus, au moins un agent diluant, en particulier la cellulose microcristalline, et au moins un lubrifiant ou un agent d'écoulement, en particulier choisi parmi le stéarate de magnésium, la silice colloïdale, et leurs mélanges.

En particulier, ces différents excipients sont mis en oeuvre dans des teneurs telles que définies précédemment.

Selon un autre mode de réalisation, une forme pharmaceutique orale solide selon l'invention, de type comprimé, peut contenir, outre les microparticules à libération modifiée d'actif, les microparticules d'agent viscosifiant et les éventuelles microparticules d'agent séquestrant :

- des diluants ou des agents de compression, comme le lactose, le saccharose, le mannitol (grades de Pearlitol® de Roquette, en particulier le Pearlitol® SD200), le xylitol, l'erythritol, les sorbitols, la cellulose microcristalline (Avicel® de FMC Biopolymer, Cellet® de Pharmatrans ou les Celphere® d'Asahi Kasei), les carbonates de calcium sous forme de cristal (Omyapure® 35 de Omya) ou sous forme de particules déjà formulées avec des agents liants (Destab® 90 S Ultra 250 de Particle Dynamics ou Hubercal® CCG4100 d'Huber), les phosphates di- et tricalciques (Dicafos® et Tricafos® de Budenheim), l'oxyde de magnésium ;
- des lubrifiants ou des agents d'écoulement comme les stéarates en particulier le stéarate de magnésium, le stéarate de calcium ou le stéarate de zinc, l'acide stéarique, le béhénate de glycérol, le stéaryle fumarate de sodium, le talc, la silice colloïdale ;
- des agents liants, comme l'hydroxyéthylcellulose, l'éthylcellulose, l'hydroxypropylcellulose (Kulcel® d'Aqualon-Hercules), l'hydroxypropylméthylcellulose (ou hypromellose) (Methocel® E ou K et en particulier Methocel® K15M de Dow), la methylcellulose (Methocel® A15 de Dow), la polyvinylpyrrolidone (ou povidone) (Plasdone® K29/32 d'ISP, Kollidon® 30 de BASF), les copolymères de vinylpyrrolidone et_d'acétate de vinyl (ou copovidone) (Plasdone® S630 d'ISP, Kollidon® VA 64 de BASF), le polyoxyde d'éthylène, les polyalkylènes glycol comme par exemple le polyéthylène glycol, les dextroses, les amidons prégélatinés, les maltodextrines, l'alcool polyvinylique, le palmitostéarate de glycérol ;
- des désintégrants, comme les amidons et amidons prégélatinés (par exemple l'amidon de maïs), la carboxyméthylcellulose, la croscarmellose, la crospovidone (grades de Polyplasdone® d'ISP, Kollidon® CL de BASF), l'hydroxypropylcellulose faiblement substituée ;
- des colorants ou pigments, comme le dioxide de titane, le sulfate de calcium, le carbonate de calcium précipité, les oxydes de fer, les colorants alimentaires naturels tels que les caramels, les caroténoïdes, le carmin, les chlorophyllines, le Rocou (ou annatto), les xanthophylles, les anthocyanes, la bétanine, l'aluminium, et les colorants alimentaires de synthèse comme les jaunes n° 5 et n° 6, les rouges n° 3 et n° 40, le vert n° 3 et le vert Emeraude, les bleus n° 1 et n° 2 ;

- des arômes, par exemple les arômes fraise, orange, banane, menthe ;
- des conservateurs, comme les parabens, en particulier le méthylparaben, l'éthylparaben, le propylparaben et le butylparaben, l'acide benzoïque et ses sels (par exemple le benzoate de sodium), le chlorocresol, l'acide sorbique et ses sels, la glycérine ;
- et leurs mélanges.

Le choix de ces excipients pour une forme solide orale de type comprimé relève clairement des compétences de l'homme de l'art.

Une forme solide selon l'invention de type comprimé peut notamment comprendre un ou plusieurs agent(s) de compression ou diluant(s) dans une teneur allant de 10 % à 80 % en poids, en particulier de 30 % à 75 % en poids, et plus particulièrement de 35 % à 65 % en poids par rapport au poids total de la forme solide.

Une forme solide selon l'invention, de type comprimé, peut comprendre un ou plusieurs lubrifiant(s) ou agent(s) d'écoulement dans une teneur allant de 0,1 % à 5 % en poids, en particulier de 0,5 % à 2 % en poids par rapport au poids total de la forme solide de type comprimé.

Selon un autre mode de réalisation particulier de l'invention, la teneur en liant(s) dans une forme solide selon l'invention de type comprimé peut aller de 0 % à 40 % en poids, en particulier de 0 % à 30 % en poids, et plus particulièrement de 5 à 20 % en poids par rapport au poids total de la forme solide.

Selon un mode de réalisation particulier, une forme solide selon l'invention de type comprimé comprend, outre les microparticules à libération modifiée d'actif, définies ci-dessus, au moins un agent de compression ou un diluant, en particulier choisi parmi la cellulose microcristalline, le mannitol et leurs mélanges, et au moins un lubrifiant ou un agent d'écoulement, en particulier choisi parmi le stéarate de magnésium, la silice colloïdale et leurs mélanges, et éventuellement au moins un liant, notamment choisi parmi l'hydroxypropylméthylcellulose et la méthylcellulose.

En particulier, ces différents excipients sont mis en oeuvre dans des teneurs telles que définies précédemment.

Selon un mode de réalisation particulier, une forme solide selon l'invention, de type gélule ou comprimé, comprend moins de 1 % en poids de désintégrant(s) par rapport à son poids total, et plus particulièrement, est dépourvue de désintégrant.

Selon encore un autre mode de réalisation particulier, une forme solide selon l'invention est dépourvue, en ce qui concernent les excipients distincts des microparticules à libération modifiée, de composé cireux insoluble dans l'eau, et en particulier est dépourvue de cires.

Les exemples et figures qui suivent sont présentés à titre illustratif du domaine de l'invention.

## FIGURES

Figure 1 : Représentation schématique d'un profil de libération modifiée d'actif comportant trois phases. En milieu aqueux acide de pH inférieur à 4,0, le début de la libération de l'actif intervient au point A, après un temps de séjour déterminé. Au point B, qui correspond à l'augmentation du pH, la libération du principe actif est accélérée.

Figure 2 : Schéma des microparticules selon l'invention avec une particule support (1), recouverte d'une couche contenant au moins un actif (2), elle-même pelliculée par un enrobage (3) contenant au moins le polymère A et le polymère B. Les proportions relatives de ces trois éléments constitutifs ne sont pas respectées sur ce schéma.

Figures 3 a et b : Schéma d'une forme solide, de type comprimé ou gélule, contenant des microparticules à libération modifiée d'actif selon l'invention.

Figure 3a : La forme solide orale comporte des microparticules à libération modifiée d'actif (1), des microparticules d'agent viscosifiant (2) et un ou des excipient(s) pharmaceutiquement acceptable(s) (3) sous la forme d'une poudre libre (dans le cas d'une gélule) ou d'une matrice solide (dans le cas d'un comprimé) dans lequel les microparticules sont dispersées.

Figure 3b : La forme solide orale comporte des microparticules à libération modifiée d'actif (1), des microparticules d'agent viscosifiant (2), des microparticules d'agent séquestrant (4) et un ou des excipient(s) pharmaceutiquement acceptable(s) (3) dans lequel les microparticules sont dispersées.

Figure 4 : Profils comparatifs de libération *in vitro*, obtenus pour des microparticules de chlorhydrate d'oxymorphone, préparées selon l'exemple 1, dans les différents milieux de dissolution HCl 0,1N et tampon phosphate à pH 4,5, pH 6,0, pH 6,8 et pH 7,4.

Figure 5 : Photos des microparticules de chlorhydrate d'oxymorphone, préparées selon l'exemple 1, intactes (5a) et broyées (5b) pendant 50 tours au mortier/au pilon, comme expliqué dans l'exemple 1.

Figure 6 : Profils comparatifs de libération *in vitro*, obtenus pour des microparticules de chlorhydrate d'oxymorphone intactes et broyées pendant 50 tours au mortier/pilon, préparées et broyées selon l'exemple 1, dans un milieu de dissolution HCl 0,1N.

Figure 7 : Profils comparatifs de libération *in vitro*, obtenus pour des comprimés de chlorhydrate d'oxymorphone préparés selon l'exemple 2, et des microparticules de chlorhydrate d'oxymorphone préparées selon l'exemple 1, lors d'exposition séquencée pendant 2 heures dans un milieu de dissolution HCl 0,1N, puis tampon phosphate à pH 7,4.

Figure 8 : Profils comparatifs de libération *in vitro*, obtenus pour des comprimés de chlorhydrate d'oxymorphone intacts et broyés pendant 50 tours au mortier/pilon, préparés et broyés selon l'exemple 2, dans un milieu de dissolution HCl 0,1N.

Figure 9 : Profils comparatifs de libération *in vitro*, obtenus pour des gélules de chlorhydrate d'oxymorphone intactes et broyées pendant 50 tours au mortier/pilon, préparées et broyées selon l'exemple 3, dans un milieu de dissolution HCl0,1N.

Figure 10 : Profils comparatifs de libération *in vitro*, obtenus pour des gélules de chlorhydrate d'oxymorphone intactes et broyées pendant 50 tours au mortier/pilon, préparées et broyées selon l'exemple 4, dans un milieu de dissolution HCl0,1N.

Figure 11 : Profil de libération *in vitro* des microparticules de chlorhydrate d'oxycodone, préparées selon l'exemple 5, lors d'une exposition séquencée pendant 2 heures dans un milieu de dissolution HCl 0,1N, puis tampon phosphate à pH 7,4.

Figure 12 : Profils comparatifs de libération *in vitro*, obtenus pour des microparticules de chlorhydrate d'oxycodone, intactes et broyées pendant 50 tours au mortier/pilon, préparées et broyées selon l'exemple 5, dans un milieu de dissolution HCl 0,1N.

Figure 13 : Profils comparatifs de libération *in vitro*, obtenus pour des microparticules de chlorhydrate d'oxycodone préparées selon l'exemple 5, et pour des gélules de chlorhydrate d'oxycodone préparées selon l'exemple 6, lors d'une exposition séquencée pendant 2 heures dans un milieu de dissolution HCl 0,1N, puis tampon phosphate à pH 7,4.

Figure 14 : Profils comparatifs de libération *in vitro*, obtenus pour des gélules de chlorhydrate d'oxycodone intactes et broyées pendant 50 tours au mortier/pilon, préparées et broyées selon l'exemple 6, dans un milieu de dissolution HCl0,1N.

Figure 15 : Profil comparatif de libération *in vitro* des microparticules de chlorhydrate d'oxycodone, présentant un taux de pelliculage de 30% et préparées selon l'exemple 7 (hors invention) dans les différents milieux de dissolution tampon phosphate à pH 6,8 et HCl 0,1N.

Figure 16 : Profils comparatifs de libération *in vitro*, obtenus pour des microparticules de chlorhydrate d'oxycodone présentant un taux de pelliculage de 30% et préparées selon l'exemple 7 (hors invention), intactes et broyées pendant 50 tours au mortier/pilon, préparées et broyées selon l'exemple 7 (hors invention), dans un milieu de dissolution HCl 0,1N.

Figure 17 : Profil de libération *in vitro* des microparticules de chlorhydrate d'oxycodone de taille strictement supérieure à 600 $\mu$m, préparées selon l'exemple 8 (hors invention), lors d'un exposition séquencée pendant 2 heures dans un milieu de dissolution HC10,1N, puis tampon phosphate à pH 7,5.

Figure 18 : Profils comparatifs de libération *in vitro*, obtenus pour des microparticules de chlorhydrate d'oxycodone de taille strictement supérieure à 600 $\mu$m, intactes et broyées pendant 50 tours au mortier/pilon, préparées et broyées selon l'exemple 8 (hors invention), dans un milieu de dissolution HCl 0,1N.

### *Exemple 1*

#### *Réparation de microparticules de chlorhydrate d'oxymorphone*

#### Phase 1 : préparation des granulés

1615 g de chlorhydrate d'oxymorphone et 85 g de povidone (Plasdone® K29/32 de ISP) sont introduits sous agitation dans un réacteur comprenant 2052,1 g d'eau et 1105,0 g d'éthanol. La solution est chauffée à 65 °C. Lorsque les cristaux de chlorhydrate d'oxymorphone et la povidone sont dissous, la solution est pulvérisée en totalité sur 300 g de sphères de cellulose (Cellet® 90 de Pharmatrans) dans un lit d'air fluidisé GPCG1.1 dans une configuration bottom spray. Après pulvérisation, le produit obtenu est tamisé sur des tamis de 80 $\mu$m et 250 $\mu$m. 1801,9 g de granulés de 80 $\mu$m à 250 $\mu$m (ce qui correspond à la fraction de produit ayant traversé les mailles du tamis de 250 $\mu$m et étant retenu sur le tamis de 80 $\mu$m) sont alors récupérés.

Phase 2 : Phase d'enrobage.

450 g de granulés obtenus lors de la phase 1 sont enrobés à température ambiante, dans un lit d'air fluidisé GPCG1.1, avec 90 g d'un copolymère d'acide méthacrylique et d'acrylate d'éthyle (Eudragit® L100-55 d'Evonik), 135 g d'un copolymère d'acide méthacrylique et de méthacrylate de méthyle (Eudragit® S100 d'Evonik), 180 g d'éthyl cellulose (Ethocel® 20 premium de Dow) et 45 g de triéthylcitrate (de Morflex) dissous dans un mélange acétone/isopropanol/eau (54/36/10 en pourcentages massiques). Après pulvérisation, les microparticules enrobées sont récupérées. Leur diamètre moyen en volume, déterminé selon la méthode détaillée ci-après, est de 270 μm

Mesure du diamètre moyen D(4;3) par diffraction laser

La distribution de tailles des particules est mesurée par diffraction laser à l'aide d'un appareil Mastersizer® 2000 de Malvern Instrument équipé d'un échantillonneur de poudre sèche de type Sirocco 2000. A partir de la répartition granulométrique mesurée sur une large gamme, le diamètre moyen équivalent en volume ou D(4;3) est calculé selon la formule suivante :

$$D(4;3) = \Sigma(d^4) / \Sigma(d^3)$$

Profils de dissolution des microparticules

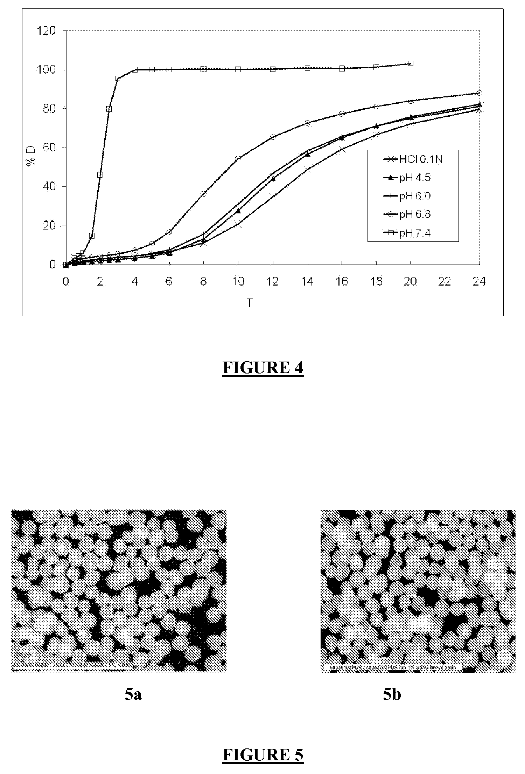

Les profils de *dissolution in vitro* des microparticules préparées ci-dessus sont mesurés par spectrométrie UV dans 900 ml des milieux de dissolution HCl à 0,1 N et tampon phosphate à pH 4,5, pH 6,0, pH 6,8 et pH 7,4, tous maintenus à 37,0 ± 0,5 °C et agités par une palette tournant à 100 rpm. Les profils de dissolution obtenus pour les microparticules dans les différents milieux sont présentés en Figure 4. Les profils de dissolution montrent une augmentation de la vitesse de libération *in vitro* de l'actif dans les milieux tampon phosphate à pH 6,8 et pH 7,4 par rapport aux vitesses de libération observées dans les milieux de dissolution HCl 0,1N, et tampon phosphate à pH 4,5 et à pH 6,0.

Broyage des microparticules

Une fraction des microparticules obtenues lors de la phase 2 et correspondant à une dose de 80 mg en chlorhydrate d'oxymorphone, soit environ 320 mg, a été broyée à l'aide d'un mortier en pyrex de 250 ml et d'un pilon en pyrex pendant 50 tours (soit environ 1 minute). Le test de broyage utilisé est détaillé ci-après.
Les photos, prises sous une loupe binoculaire, des microparticules avant et après broyage sont montrées respectivement sur les Figures 5a et 5b.
L'aspect des microparticules, observé sous une loupe binoculaire, est le même avant et après broyage.

Test de broyage

Comme évoqué précédemment, la technique du mortier/pilon est utilisée comme test de broyage pour déterminer la résistance au broyage des microparticules d'actif ou de la forme pharmaceutique solide orale contenant les microparticules d'actif selon l'invention.
Une dose de principe actif sous la forme de microparticules ou d'une forme pharmaceutique orale intacte (un comprimé ou le contenu d'une gélule) est introduite dans un mortier en pyrex de 250 ml et broyée à l'aide du pilon en pyrex correspondant au mortier pendant 50 tours, soit pendant environ 1 minute.
La vitesse de libération *in vitro* du principe actif contenu dans la poudre obtenue après broyage est alors déterminée au cours d'un test de dissolution. Ce test, identique au test de dissolution des microparticules ou de la forme pharmaceutique orale intacte, est réalisé selon la méthode de la Pharmacopée Européenne 6ième Edition 6.5 chapitre 2.9.3 - Essai de dissolution des formes solides.
Les profils de dissolution des microparticules intactes et broyées ou de la forme pharmaceutique orale intacte et broyée sont comparés : la différence à 0,5 heure entre le profil de dissolution de la poudre broyée et celui de la formulation intacte correspond à la proportion de microparticules ou de la forme pharmaceutique orale détériorée(s) après broyage, la proportion restante correspondant à la proportion de microparticules ou de la forme pharmaceutique orale selon l'invention qui a (ont) résisté au broyage.

Profils de dissolution des particules intactes et broyées

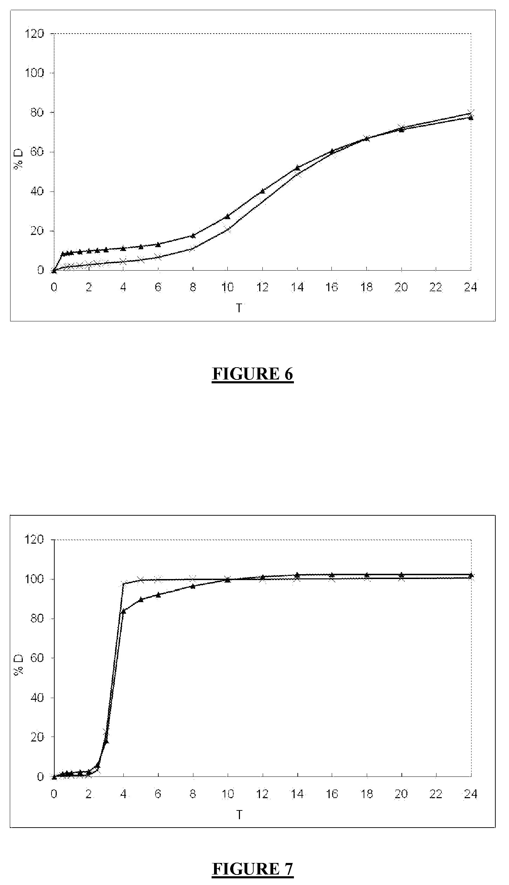

Les profils de *dissolution in vitro* des microparticules intactes, préparées ci-dessus, et des mêmes microparticules broyées, sont mesurés par spectrométrie UV dans 900 ml d'un milieu de dissolution HCl à 0,1 N maintenus à 37,0 ± 0,5 °C et agités par une palette tournant à 100 rpm. Les profils de dissolution obtenus pour les microparticules intactes (✳) et broyées (▲) sont comparés en Figure 6. Les deux profils de dissolution sont similaires et présentent un facteur de similarité selon la pharmacopée européenne de 62 %. On constate que moins de 10 % des microparticules ont été endommagées. Les microparticules ayant subi le broyage ont conservé leurs propriétés de libération modifiée.

***Exemple 2***

*Réparation de comprimés de chlorhydrate d'oxymorphone*

Préparation des comprimés

11,0 g des microparticules à libération différée et modifiée préparées dans l'exemple précédente (phase 2), sont mélangés à 8,0 g de polyoxyéthylène (Sentry Polyox WSR® 303 de Dow), préalablement tamisés sur des tamis de 150 μm et de 300 μm, la fraction conservée étant celle dont la taille est comprise entre 150 μm et 300 μm) 2,0 g d'hypromellose (Methocel® K15M EP de Colorcon), 12,0 g de méthyl cellulose (Methocel® A15 LV de Colorcon), 24,0 g de cellulose microcristalline (Avicel® PH301 de FMC), 24,0 g de mannitol (Pearlitol® SD 200 de Roquette), 40 g de sphères de cellulose (de Asahi Kasei) et 1,0 g de stéarate de magnésium. Ce mélange est utilisé pour la fabrication de comprimés ronds de diamètre 12 mm de 611 mg, grâce à une presse XP1 de Korsch. La force de compression appliquée sur le mélange est de 25 kN. Les comprimés ainsi fabriqués ont une dureté d'environ 97 N.

Profils de dissolution dans des conditions d'expositions séquentielles

Le profil de dissolution *in vitro* du comprimé préparé ci-dessus, est mesuré par spectrométrie UV dans 900 ml d'un milieu de dissolution HCl à 0,1 N maintenus à 37,0 ± 0,5 °C et agités par une palette tournant à 100 rpm pendant 2 heures puis, après ajustement du pH et de la salinité du milieu, à tampon phosphate de pH 7,4 et à 0,05 M de phosphate de potassium.

Le profil de dissolution du comprimé obtenu (▲) est comparé en Figure 7 au profil des microparticules intactes (✳) préparées selon l'exemple 1. Les deux profils de dissolution sont similaires et présentent un facteur de similarité selon la pharmacopée européenne de 58 %.

Broyage des comprimés

Un comprimé obtenu selon l'exemple 2, correspondant à une dose de 20 mg en chlorhydrate d'oxymorphone, a été broyé à l'aide d'un mortier en pyrex de 250 ml et d'un pilon en pyrex pendant 50 tours (soit environ 1 minute). Le test de broyage utilisé est celui décrit précédemment.

Profils de dissolution des comprimés intacts et broyés

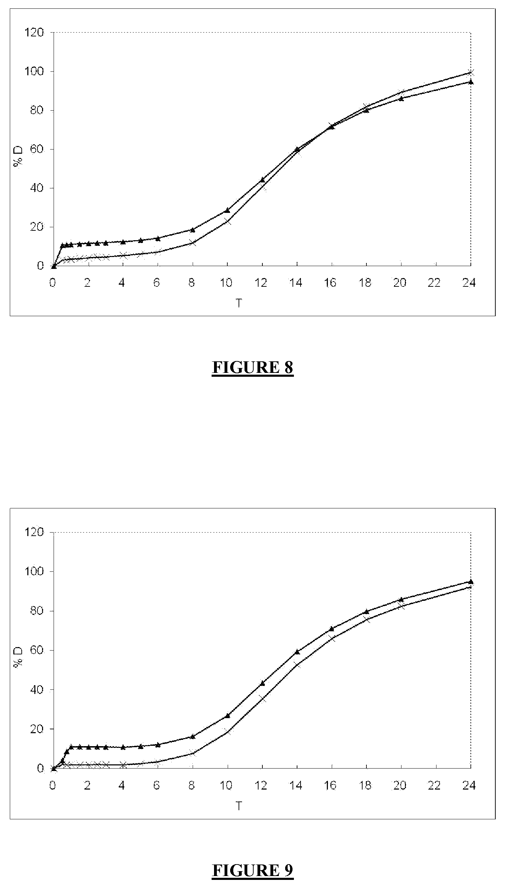

Les profils de *dissolution in vitro* des comprimés intacts, préparés ci-dessus, et des mêmes comprimés broyés, sont mesurés par spectrométrie UV dans 900 ml de HCl à 0,1 N maintenus à 37,0 ± 0,5 °C et agités par une palette tournant à 100 rpm. Les profils de dissolution obtenus pour les comprimés intacts (✳) et broyés (▲) sont comparés en Figure 8. Environ 10 % des microparticules ont été endommagées. Les autres microparticules ont conservé leur profil de libération modifiée. Les deux profils de dissolution sont similaires et présentent un facteur de similarité selon la pharmacopée européenne de 61 %.

*Test in vitro* d'extraction pour injection

Un comprimé de chlorhydrate d'oxymorphone préparé ci-dessus est broyé à l'aide d'un mortier en pyrex de 250 ml et d'un pilon en pyrex pendant 50 tours (soit environ 1 minute). 10 ml d'eau du robinet sont versés sur la poudre. La dispersion est ensuite agitée pendant 10 minutes à l'aide d'un barreau aimanté. La dispersion est alors soutirée pendant 5 minutes par une seringue de 10 ml, à travers une aiguille 27G dont la pointe est recouverte d'une boulette de coton.

La quantité de liquide soutirée dans la seringue est inférieure à 0,1 ml, correspondant à moins de 1 % du volume de solvant d'extraction introduit.

### *Exemple 3*

*Réparation de gélules de chlorhydrate d'oxymorphone*

Préparation des gélules

13,8 g des microparticules à libération modifiée préparées dans l'exemple 1 (phase 2), sont mélangés à 10,0 g de polyoxyéthylène (Sentry Polyox WSR® 303 de Dow, préalablement tamisés sur des tamis de 150 $\mu$m et de 300 $\mu$m, la fraction conservée étant celle dont la taille est comprise entre 150 $\mu$m et 300 $\mu$m), 50,0 g de sphères de cellulose (de Asahi Kasei), 0.8 g de silice colloïdale (Aerosil® 200 de Evonik) et 0,4 g de stéarate de magnésium. Ce mélange est utilisé pour la fabrication de gélules de taille 0 contenant 300 mg de mélange soit une dose de 20 mg de chlorhydrate d'oxymorphone.

Broyage des gélules

Le contenu d'une gélule obtenue selon l'exemple 3 a été broyé à l'aide d'un mortier en pyrex de 250 ml et d'un pilon en pyrex pendant 50 tours (soit environ 1 minute).

Profils de dissolution des gélules intactes et broyées

Les profils de *dissolution in vitro* des gélules intactes, préparées ci-dessus, et du contenu broyé des mêmes gélules, sont mesurés par spectrométrie UV dans 900 ml d'un milieu de dissolution HCl à 0,1 N maintenus à 37,0 $\pm$ 0,5 °C et agités par une palette tournant à 100 rpm. Les profils de dissolution obtenus pour les gélules intactes ($\times$) et pour le contenu broyé des gélules ($\blacktriangle$) sont comparés en Figure 9. Environ 10 % des microparticules ont été endommagées. Les autres microparticules ont conservé leur profil de libération modifiée. Les deux profils de dissolution sont similaires et présentent un facteur de similarité selon la pharmacopée européenne de 56 %.

*Test in vitro* d'extraction pour injection

Le contenu d'une gélule de chlorhydrate d'oxymorphone obtenue selon l'exemple 3 est broyé à l'aide d'un mortier en pyrex de 250 ml et d'un pilon en pyrex pendant 50 tours (soit environ 1 minute). 10 ml d'eau du robinet sont versés sur la poudre broyée. La dispersion est ensuite agitée pendant 10 minutes à l'aide d'un barreau aimanté. La dispersion est alors soutirée pendant 5 minutes par une seringue de 10 ml, à travers une aiguille 27G dont la pointe est recouverte d'une boulette de coton.
La quantité de liquide soutirée dans la seringue est inférieure à 0,6 ml, correspondant à moins de 6% du volume de solvant d'extraction introduit.

### *Exemple 4*

*Réparation de gélules de chlorhydrate d'oxymorphone*

Préparation des gélules

13,8 g des microparticules à libération modifiée préparées dans l'exemple 1 (phase 2), sont mélangés à 10,0 g de polyoxyéthylène (Sentry Polyox WSR® 303 de Dow, préalablement tamisés sur des tamis de 150 $\mu$m et de 300 $\mu$m, la fraction conservée étant celle dont la taille est comprise entre 150 $\mu$m et 300 $\mu$m), 25,0 g de résine échangeuse de cations (Amberlite® IR69F de Rohm & Haas préalablement séchée, broyée et tamisée sur des tamis de 150 $\mu$m et de 300 $\mu$m, la fraction conservée étant celle dont la taille est comprise entre 150 $\mu$m et 300 $\mu$m), 50,0 g de sphères de cellulose (de Asahi Kasei), 1,0 g de silice colloïdale (Aerosil® 200 de Evonik) et 0,5 g de stéarate de magnésium. Ce mélange est utilisé pour la fabrication de gélules de taille 0 contenant 401 mg de mélange soit une dose de 20 mg de chlorhydrate d'oxymorphone.

Broyage des gélules

Le contenu d'une gélule obtenue selon l'exemple 4 a été broyé à l'aide d'un mortier en pyrex de 250 ml et d'un pilon en pyrex pendant 50 tours (soit environ 1 minute).

Profils de dissolution des gélules intactes et broyées

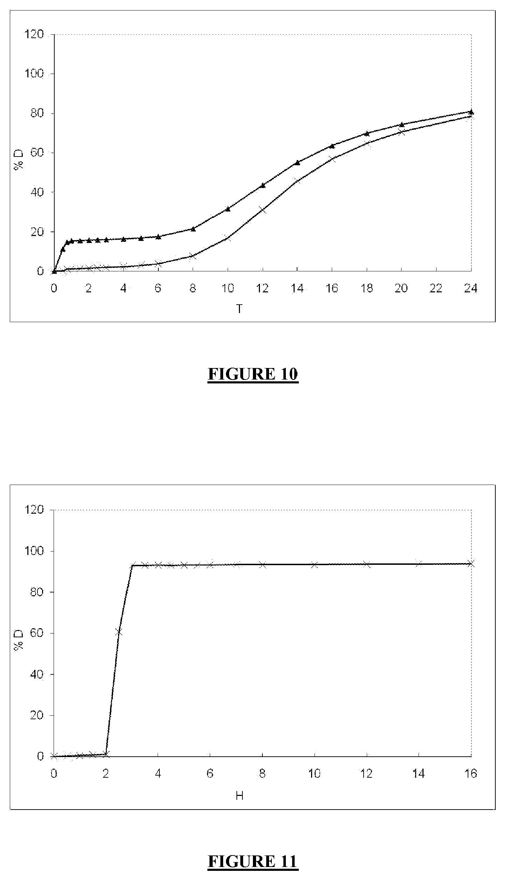

Les profils de *dissolution in vitro* des gélules intactes, préparées ci-dessus, et du contenu broyé des mêmes gélules, sont mesurés par spectrométrie UV dans 900 ml d'un milieu de dissolution HCl à 0,1 N maintenus à $37,0 \pm 0,5$ °C et agités par une palette tournant à 100 rpm. Les profils de dissolution obtenus pour les gélules intactes (✳) et pour le contenu broyé des gélules (▲) sont comparés en Figure 10. Seulement 15 % de la dose de chlorhydrate d'oxymorphone contenue dans les gélules est disponible immédiatement. Les autres microparticules sont demeurées intactes et ont conservé leur profil de libération modifiée.

Test *in vitro* d'extraction pour injection

Le contenu d'une gélule de chlorhydrate d'oxymorphone obtenue selon l'exemple 4 est broyé à l'aide d'un mortier en pyrex de 250 ml et d'un pilon en pyrex pendant 50 tours (soit environ 1 minute). 10 ml d'eau du robinet sont versés sur la poudre broyée. La dispersion est ensuite agitée pendant 10 minutes à l'aide d'un barreau aimanté. La dispersion est alors soutirée pendant 5 minutes par une seringue de 10 ml, à travers une aiguille 27G dont la pointe est recouverte d'une boulette de coton.
La quantité de liquide soutirée dans la seringue est inférieure à 0,6 ml, correspondant à moins de 6 % du volume de solvant d'extraction introduit.

Test *in vitro* d'extraction pour ingestion orale

Le contenu d'une gélule obtenue selon l'exemple 4, correspondant à une dose de 20 mg en chlorhydrate d'oxymorphone, a été broyé à l'aide d'un mortier en pyrex de 250 ml et d'un pilon en pyrex pendant 50 tours (soit environ 1 minute). La poudre broyée est récupérée et introduite dans un flacon en polyéthylène de 125 ml dans lequel sont versés 100 ml d'eau du robinet. La dispersion contenue dans le flacon en polyéthylène fermé par un bouchon vissé est agitée pendant 7 heures à température ambiante à l'aide d'un disque à rotation incliné à 45 °C et à une vitesse de rotation de 30 tours/min puis laissée au repos dans le flacon en polyéthylène fermé à température ambiante. Deux prélèvements de 3 ml de la dispersion sont effectués après 7 heures et après 24 heures de mise en contact de la poudre broyée avec les 100 ml de liquide d'extraction, puis sont filtrés sur des filtres Acrodisc® de 0,45 $\mu$m et sont analysés par chromatographie HPLC.
Les proportions de chlorhydrate d'oxymorphone dissoutes dans le liquide d'extraction (eau du robinet) par rapport aux 20 mg de chlorhydrate d'oxymorphone broyés et introduits dans le 100 ml de liquide d'extraction sont présentées dans le tableau ci-dessous.
Après 24 heures de dispersion du contenu de la gélule, préparé et broyé selon les étapes précédentes, seulement 12 % de la dose de chlorhydrate d'oxymorphone contenu dans la gélule (20 mg), soit 2,4 mg de chlorhydrate d'oxymorphone, sont disponibles immédiatement.

| Temps du Prélèvement | Proportions de chlorhydrate d'oxymorphone dissoutes dans le liquide d'extraction par rapport à la dose introduite |
|---|---|
| 7 heures | 8,1 % |
| 24 heures | 12,0 % |

### *Exemple 5*

*Réparation de microparticules* de *chlorhydrate d'oxycodone*

Phase 1 : Préparation des granulés

1615,0 g de chlorhydrate d'oxycodone et 85,0 g de povidone (Plasdone® K29/32 de ISP) sont introduits sous agitation dans un réacteur comprenant 2052,1 g d'eau et 1105,0 g d'éthanol. La solution est chauffée à 65 °C. Lorsque les cristaux de chlorhydrate d'oxycodone et la povidone sont dissous, la solution est pulvérisée en totalité sur 300,0 g de sphères de cellulose (Cellet® 90 de Pharmatrans) dans un lit d'air fluidisé GPCG1.1 dans une configuration bottom spray.
En fin de pulvérisation, le produit obtenu est tamisé sur des tamis de 80 $\mu$m et 250 $\mu$m 2054,6 g de granulés

de 80 μm à 250 μm (ce qui correspond à la fraction de produit ayant traversé les mailles du tamis de 250 μm et étant retenu sur le tamis de 80 μm) sont alors récupérés.

Phase 2 : Phase d'enrobage.

400,0 g de granulés obtenus lors de la phase 1 sont enrobés à température ambiante, dans un lit d'air fluidisé GPCG1.1, avec 119,99 g d'un copolymère d'acide méthacrylique et d'acrylate d'éthyle (Eudragit® L100-55 d'Evonik), 80,01 g d'un copolymère d'acide méthacrylique et de méthacrylate de méthyle (Eudragit® S100 d'Evonik), 160,02 g d'éthyl cellulose (Ethocel® 20 premium de Dow) et 40,02 g de triéthylcitrate (Citrofol AI de Jungbunzlauer) dissous dans un mélange de 2484,0 g d'acétone, 1656,0 g d'isopropanol et 460,0 g d'eau.

Après pulvérisation de 3333 g de solution de pelliculage, un prélèvement de 11,5 g de particules est effectué. Le taux de pelliculage des microparticules prélevées est de 40 %. Le diamètre moyen en volume des microparticules prélevées, déterminé par diffraction laser selon la méthode décrite précédemment, est de 275 μm.

Profils de dissolution dans des conditions d'expositions séquentielles

Le profil de dissolution *in vitro* des microparticules de chlorhydrate d'oxycodone, préparées ci-dessus, est mesuré par spectrométrie UV dans 900 ml de HCl à 0,1 N pendant 2 heures, puis après ajustement du pH et de la salinité du milieu, à pH 7,4 et à 0,05M de phosphate de potassium, maintenus à 37,0 ± 0,5 °C et agités par une palette tournant à 100 rpm.

Le profil de dissolution obtenu est présenté en Figure 11. Les microparticules de chlorhydrate d'oxycodone préparées montrent un profil de libération dépendant du temps et du pH du milieu environnant.

Broyage des microparticules

La quantité de microparticules obtenues lors de la phase 2 et correspondant à une dose de 80 mg en chlorhydrate d'oxycodone, soit environ 175 mg, a été broyée à l'aide d'un mortier en pyrex de 250 ml et d'un pilon en pyrex pendant 50 tours (soit environ 1 minute). Le test de broyage utilisé est celui décrit précédemment.

Profils de dissolution des particules intactes et broyées

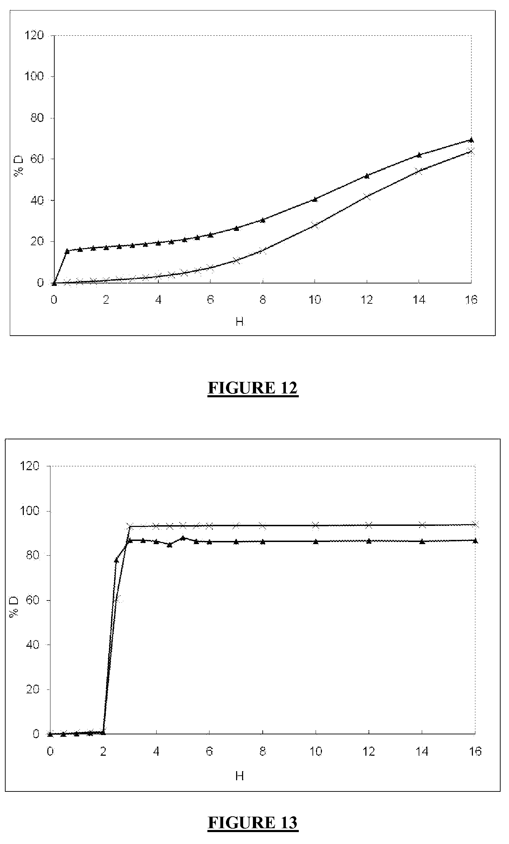

Les profils de dissolution *in vitro* des microparticules intactes, préparées ci-dessus à la phase 2, et des mêmes microparticules broyées, sont mesurés par spectrométrie UV dans 900 ml de HCl à 0,1 N maintenus à 37,0 ± 0,5 °C et agités par une palette tournant à 100 rpm. Les profils de dissolution obtenus pour les microparticules intactes (✳) et broyées (▲) sont comparés en Figure 12.

On constate que seulement 15 % des microparticules ont été endommagées lors du broyage. Les autres microparticules ont conservé intactes leurs propriétés de libération modifiée.

## *Exemple 6*

*Réparation de gélules de chlorhydrate d'oxycodone*

Préparation des gélules

1,730 g des microparticules à libération modifiée préparées dans l'exemple 5 (phase 2), sont mélangés à 0,400 g de polyoxyéthylène (Sentry Polyox WSR® 303 de Dow, préalablement tamisés sur des tamis de 150 μm et de 300 μm, la fraction conservée étant celle dont la taille est comprise entre 150 μm et 300 μm), 1,007 g de résine échangeuse de cations (Amberlité IR69F de Rohm & Haas préalablement séchée, broyée et tamisée sur des tamis de 150 μm et de 300 μm, la fraction conservée étant celle dont la taille est comprise entre 150 μm et 300 μm), 0,035 g de silice colloïdale (Aerosil® 200 de Evonik) et 0,016 g de stéarate de magnésium. Ce mélange est utilisé pour la fabrication de gélules de taille 0 contenant 319 mg de mélange soit une dose de 80 mg de chlorhydrate d'oxycodone.

Profils de dissolution dans des conditions d'expositions séquentielles

Le profil de dissolution *in vitro* des gélules de chlorhydrate d'oxycodone, préparées ci-dessus, est mesuré par spectrométrie UV dans 900 ml de HCl à 0,1 N pendant 2 heures puis, après ajustement du pH et de la salinité du milieu, à pH 7,5 et à 0,05M de phosphate de potassium, maintenus à 37,0 ± 0,5 °C et agités par une palette tournant à 100 rpm.

Le profil de dissolution des gélules obtenues (▲) est comparé en Figure 13 au profil des microparticules intactes (✳) préparées selon l'exemple 5. Les deux profils de dissolution sont similaires et présentent un facteur de similarité selon la pharmacopée européenne de 58 %.

Broyage des gélules

Le contenu d'une gélule obtenue ci-dessus a été broyé à l'aide d'un mortier en pyrex de 250 ml et d'un pilon en pyrex pendant 50 tours (soit environ 1 minute).

Profils de dissolution des gélules intactes et broyées

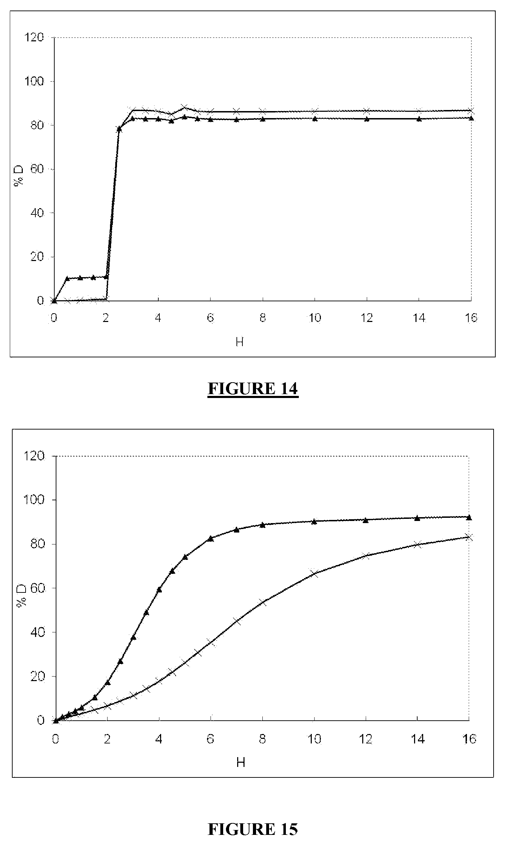

Les profils de dissolution *in vitro* des gélules intactes, préparées ci-dessus, et du contenu broyé des mêmes gélules sont mesurés par spectrométrie UV dans 900 ml de HCl à 0,1 N pendant 2 heures puis, après ajustement du pH et de la salinité du milieu, à pH 7,4 et à 0,05M de phosphate de potassium, maintenus à 37,0 ± 0,5 °C et agités par une palette tournant à 100 rpm. Les profils de dissolution obtenus pour les gélules intactes (✳) et pour le contenu broyé des gélules (▲) sont comparés en Figure 14.
On constate que seulement 10% des microparticules ont été endommagés lors du broyage. Les autres microparticules ont conservé leur profil de libération modifiée.

*Test in vitro* d'extraction pour injection

Le contenu d'une gélule de chlorhydrate d'oxycodone obtenue selon l'exemple 6 est broyé à l'aide d'un mortier en pyrex de 250 ml et d'un pilon en pyrex pendant 50 tours (soit environ 1 minute). 10 ml d'eau du robinet sont versés sur la poudre broyée. La dispersion est ensuite agitée pendant 10 minutes à l'aide d'un barreau aimanté. La dispersion est alors soutirée pendant 5 minutes par une seringue de 10 ml, à travers une aiguille 27G dont la pointe est recouverte d'une boulette de coton.
La quantité de liquide soutirée dans la seringue est égale à 0,2 ml, correspondant 2% du volume de solvant d'extraction introduit.

**Exemple 7 (Hors invention)**

*Préparation de microparticules de chlorhydrate d'oxycodone présentant un taux de pelliculage de 30 %*

2143 g de solution de pelliculage obtenue selon la phase 2 de l'exemple 5, sont pulvérisés sur 400,0 g de granulés obtenus selon la phase 1 de l'exemple 5. Un prélèvement de 9,0 g de particules est effectué. Le taux de pelliculage des microparticules prélevées est de 30 %. Le diamètre moyen en volume des microparticules prélevées est de 263 μm.

Profils de dissolution des microparticules

Le profil de dissolution *in vitro* des microparticules de chlorhydrate d'oxycodone, préparés ci-dessus, est mesuré par spectrométrie UV dans 900 ml de HCl à 0,1 N et dans 900 ml de tampon à 0,05M de phosphate de potassium à pH 6,8, maintenus à 37,0 ± 0,5 °C et agités par une palette tournant à 100 rpm. Les profils de dissolution obtenus sont présentés en Figure 15.
Les microparticules de chlorhydrate d'oxycodone préparées avec un taux de pelliculage de 30 % ont bien un profil de libération accéléré dans le milieu à pH 6,8 (▲) par rapport à celui obtenu dans HCl 0,1N (✳).

Broyage des microparticules

Environ 142 mg de microparticules préparées avec un taux de pelliculage de 30%, quantités correspondant à une dose de 80 mg en chlorhydrate d'oxycodone, ont été broyés à l'aide d'un mortier en pyrex de 250 ml et d'un pilon en pyrex pendant 50 tours (soit environ 1 minute).

Profils de dissolution des particules intactes et broyées

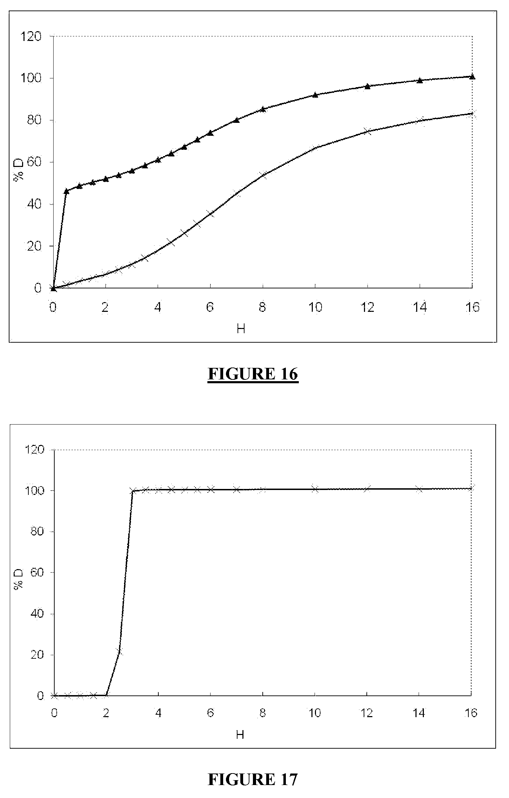

Les profils de dissolution *in vitro* des microparticules intactes, préparées ci-dessus, et des mêmes microparticules broyées, sont mesurés par spectrométrie UV dans 900 ml de HCl à 0,1 N maintenus à 37,0 ± 0,5 °C et agités par une palette tournant à 100 rpm. Les profils de dissolution obtenus pour les microparticules intactes (✳) et broyées (▲) sont comparés en Figure 16,

On constate que 67 % des microparticules ont été endommagées lors du broyage et n'ont pas conservé leurs propriétés de libération modifiée.

### *Exemple 8 (Hors invention)*

### *Réparation de microparticules de chlorhydrate d'oxycodone de taille strictement supérieure à 600 $\mu$m*

### Phase 1 : Préparation des granulés

137,3 g de chlorhydrate d'oxycodone et 7,2 g de povidone (Plasdone® K29/32 de ISP) sont introduits sous agitation dans un réacteur comprenant 174,4 g d'eau et 93,9 g d'éthanol. La solution est chauffée à 65 °C. Lorsque les cristaux de chlorhydrate d'oxycodone et la povidone sont dissous, la solution est pulvérisée en totalité sur 25,5 g de sphères de cellulose (Celphere CP203 d'Asahi Kasei) dans un lit d'air fluidisé MinGlatt 8008 dans une configuration bottom spray.

Après pulvérisation, 157,1 g de granulés sont récupérés.

### Phase 2 : Phase d'enrobage.

40,0 g de granulés obtenus lors de la phase 1 sont enrobés à température ambiante, dans un lit d'air fluidisé GPCG1.1, avec 14,67 g d'un copolymère d'acide méthacrylique et d'acrylate d'éthyle (Eudragit® L100-55 d'Evonik), 2,60 g d'un copolymère d'acide méthacrylique et de méthacrylate de méthyle (Eudragit® S100 d'Evonik), 6,66 g d'éthyl cellulose (Ethocel® 20 premium de Dow) et 2,67 g de triéthylcitrate (Citrofol AI de Jungbunzlauer) dissous dans un mélange de 165,6 g d'acétone, 110,4 g d'isopropanol et 30,70 g d'eau.

En fin de pulvérisation, les microparticules enrobées sont récupérées. Le diamètre moyen en volume des microparticules récupérées est de 666 $\mu$m.

### Profils de dissolution dans des conditions d'expositions séquentielles

Le profil de dissolution *in vitro* des microparticules de chlorhydrate d'oxycodone, préparées ci-dessus, est mesuré par spectrométrie UV dans 900 ml de HCl à 0,1 N pendant 2 heures, puis après ajustement du pH et de la salinité du milieu, à pH 7,5 et à 0,05M de phosphate de potassium, maintenus à 37,0 $\pm$ 0,5 °C et agités par une palette tournant à 100 rpm.

Le profil de dissolution obtenu est présenté en Figure 17. Les microparticules de chlorhydrate d'oxycodone de taille supérieure à 600 $\mu$m montrent un profil de libération dépendant du temps et du pH du milieu environnant.

### Broyage des microparticules

Environ 174 mg de microparticules préparées lors de la phase 2 et correspondant à une dose de 80 mg en chlorhydrate d'oxycodone, ont été broyés à l'aide d'un mortier en pyrex de 250 ml et d'un pilon en pyrex pendant 50 tours (soit environ 1 minute).

### Profils de dissolution des particules intactes et broyées

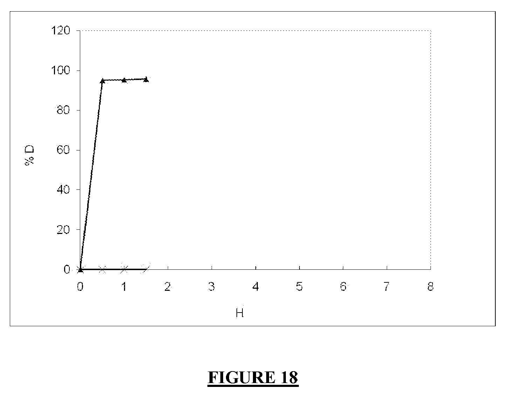

Les profils de *dissolution in vitro* des microparticules intactes, préparées ci-dessus à la phase 2, et des mêmes microparticules broyées, sont mesurés par spectrométrie UV dans 900 ml de HCl à 0,1 N maintenus à 37,0 $\pm$ 0,5 °C et agités par une palette tournant à 100 rpm. Les profils de dissolution obtenus pour les microparticules intactes (✳) et broyées (▲) sont comparés en Figure 18.

Dès les premiers temps de prélèvement du test de dissolution, soit dès 30 minutes dans le milieu acide, les microparticules broyées ont libéré 100% de la dose de chlorhydrate d'oxycodone contenue initialement dans les microparticules.

L'ensemble des microparticules de taille supérieure à 600 $\mu$m a été abîmée lors du broyage. Elles n'ont pas conservé leur propriété de libération contrôlée après broyage.

**Revendications**

1. Forme pharmaceutique solide orale à libération modifiée d'au moins un principe actif, contenant au moins des microparticules contenant ledit actif et au moins un agent viscosifiant sous une forme isolée desdites microparticules d'actif, **caractérisée en ce que** :

   - lesdites microparticules d'actif possèdent un diamètre moyen allant de 100 à 600 $\mu$m, et sont formées d'un coeur contenant au moins ledit principe actif et enrobé d'au moins une couche d'enrobage,
   - ledit coeur est formé d'une particule support recouverte d'une couche comprenant au moins ledit actif,
   - ladite couche d'enrobage est formée d'un matériau composé d'au moins :

      - 25 à 70 % en poids par rapport au poids total dudit enrobage d'au moins un polymère A insoluble dans l'eau,
      - 30 à 75 % en poids par rapport au poids total dudit enrobage d'au moins un polymère B insoluble dans l'eau en dessous de pH 5 et soluble dans l'eau au dessus de pH 7, et
      - 0 à 25 % en poids par rapport au poids total dudit enrobage d'au moins un plastifiant,

   lesdits polymères A et B sont présents dans un rapport pondéral polymère(s) B/polymère(s) A compris entre 0,75 et 4, et

      - ladite couche d'enrobage représente au moins 35 % en poids, par rapport au poids total de ladite microparticule,
      - lesdites microparticules de principe actif ont un profil *in vitro* de libération **caractérisé par** :

         - dans un milieu aqueux acide de pH inférieur à 4, un temps de latence inférieur à 12 heures, en particulier entre 0,5 et 8 heures, voire entre 1 et 5 heures, suivi par une libération prolongée du principe actif,
         - après une augmentation de pH à un pH supérieur à 7, une accélération de la libération du principe actif.

2. Forme solide selon la revendication 1, dans laquelle la couche d'enrobage desdites microparticules d'actif comprend moins de 30 % en poids de talc, par rapport au poids total de ladite couche d'enrobage, de préférence moins de 20 % en poids, notamment moins de 10 % en poids, en particulier moins de 5 % en poids, voire est exempte de talc.

3. Forme solide selon l'une quelconque des revendications précédentes, dans laquelle l'enrobage des microparticules est composé d'une unique couche formée dudit matériau.

4. Forme solide selon l'une quelconque des revendications précédentes, dans laquelle l'enrobage, disposé en surface des microparticules, est présent à un taux d'enrobage allant de 35 à 60 % en poids, en particulier de 40 à 55 % en poids, plus particulièrement de 45 à 55 % en poids par rapport au poids total de ladite microparticules.

5. Forme solide selon l'une quelconque des revendications précédentes, dans laquelle le polymère A est choisi parmi l'éthylcellulose, l'acétate butyrate de cellulose, l'acétate de cellulose, les copolymères d'ammonio (méth)acrylate, les esters d'acides poly(méth)acryliques, et leurs mélanges.

6. Forme solide selon l'une quelconque des revendications précédentes, dans laquelle l'enrobage des microparticules contient de 30 à 65 % en poids, en particulier de 35 à 60 % en poids, en particulier de 35 à 55 % en poids, en particulier de 35 à 50 % en poids de polymère(s) A par rapport à son poids total.

7. Forme solide selon l'une quelconque des revendications précédentes, dans laquelle le polymère B est choisi parmi le(s) copolymère(s) d'acide méthacrylique et de méthacrylate de méthyle, le(s) copolymère(s) d'acide méthacrylique et d'acrylate d'éthyle, l'acétate phtalate de cellulose, l'acétate succinate de cellulose, l'acétate trimellilate de cellulose, le phtalate d'hydroxypropylméthylcellulose, l'acétate succinate d'hydroxypropylméthylcellulose, la carboxyméthylcellulose, la gomme shellac, l'acétate phtalate de polyvinyle, et leurs mélanges.

8. Forme solide selon l'une quelconque des revendications précédentes, dans laquelle le polymère B est choisi parmi le(s) copolymère(s) d'acide méthacrylique et de méthacrylate de méthyle, le(s) copolymère(s) d'acide méthacrylique et d'acrylate d'éthyle, et leurs mélanges.

9. Forme solide selon l'une quelconque des revendications précédentes, dans laquelle l'enrobage des microparticules contient de 30 à 70 % en poids, en particulier de 35 à 65 % en poids, notamment de 35 % à 60 % en poids de polymère(s) B par rapport à son poids total.

**10.** Forme solide selon l'une quelconque des revendications précédentes, dans laquelle l'enrobage des microparticules est formé d'au moins un mélange comprenant, à titre de polymère A, au moins l'éthylcellulose ou l'acétate butyrate de cellulose ou le copolymère d'ammonio (méth)acrylate ou un de leurs mélanges, avec, à titre de polymère B, au moins un copolymère d'acide méthacrylique et d'acrylate d'éthyle ou un copolymère d'acide méthacrylique et de méthacrylate de méthyle ou un de leurs mélanges.

**11.** Forme solide selon l'une quelconque des revendications précédentes, dans laquelle l'enrobage comprend les polymères A et B dans un rapport pondéral polymère(s) B/polymère(s) A compris entre 0,75 et 2, et plus particulièrement entre 0,75 et 1,5.

**12.** Forme solide selon l'une quelconque des revendications précédentes, dans laquelle ladite particule support possède un diamètre moyen inférieur ou égal à 300 $\mu$m, de préférence compris entre 50 et 250 $\mu$m, plus particulièrement entre 70 et 150 $\mu$m.

**13.** Forme solide selon l'une quelconque des revendications précédentes, dans laquelle lesdites microparticules enrobées possèdent un diamètre moyen allant de 150 à 350 $\mu$m, plus particulièrement de 200 à 300 $\mu$m, en particulier de 250 à 300 $\mu$m.

**14.** Forme solide selon l'une quelconque des revendications précédentes, **caractérisée en ce que** l'agent viscosifiant est choisi parmi :

- les polyacides acryliques, en particulier les carbomer,
- les polyalkylènes glycols, par exemple les polyéthylènes glycol,
- les polyoxydes d'alkylène, par exemple les polyoxydes d'éthylène ou polyoxyéthylènes,
- les polyvinylpyrrolidones,
- les gélatines,
- les polysaccharides, de préférence choisi parmi l'alginate de sodium, les pectines, la gomme guar, les xanthanes, les carraghénanes, les gellanes, l'hydroxypropylcellulose, l'hydroxypropylméthylcellulose, la méthylcellulose, l'hydroxyéthylcellulose et la carboxyméthylcellulose,
- et leurs mélanges,

en particulier, l'agent viscosifiant est un polyoxyéthylène, en particulier possédant un haut poids moléculaire, et plus particulièrement ayant un poids moléculaire moyen allant de 1 million g/mole à environ 8 millions g/mole.

**15.** Forme solide selon l'une quelconque des revendications précédentes, dans laquelle l'actif est choisi parmi les amphétamines, les analgésiques, les anorexigènes, les antidépresseurs, les antiépileptiques, les antiparkinsoniens, les anxiolytiques, les barbituriques, les benzodiazépines, les hypnotiques, les narcotiques en particulier les opioïdes, les neuroleptiques, les psycho stimulants et les psychotropes, en particulier est un narcotique, plus particulièrement choisi parmi l'oxycodone, l'oxymorphone, l'hydromorphone, l'hydrocodone, le tramadol et leurs sels pharmaceutiquement acceptables.

**16.** Forme solide selon l'une quelconque des revendications précédentes, **caractérisée en ce qu'**elle se présente sous forme d'un comprimé ou d'une gélule.

**17.** Forme solide selon l'une quelconque des revendications précédentes, **caractérisée en ce qu'**elle comprend au moins un agent séquestrant sous forme de microparticules distinctes des microparticules d'actif, ledit agent séquestrant étant plus particulièrement choisi parmi :

- le sodium dodécyl sulfate ou le docusate de sodium ;
- les sels d'ammoniums quaternaires, en particulier le bromure de triméthyl tétradécyl ammonium ou le chlorure de benzéthonium ;
- les résines fortement acides échangeuses de cations, lorsque l'actif en solution est cationique, ou les résines fortement basiques échangeuses d'anions lorsque l'actif en solution est anionique, selon la polarité de l'actif, et leurs mélanges,

et en particulier, lorsque l'actif en solution est sous forme cationique, parmi :

- les résines échangeuses de cations, fortement acides, telles que les copolymères de styrène et de divinylbenzène sulfoniques, et
- les résines échangeuses de cations, faiblement acides, telles que les copolymères réticulés d'acide méthacrylique et de divinylbenzène ou leurs sels.

**Patentansprüche**

1. Orale feste pharmazeutische Form zur modifizierten Freisetzung mindestens eines Wirkstoffs, enthaltend mindestens Mikroteilchen, die den Wirkstoff enthalten, und mindestens ein viskositätserhöhendes Mittel in einer von den Wirkstoff-Mikroteilchen isolierten Form, **dadurch gekennzeichnet, dass**:

   - die Wirkstoff-Mikroteilchen einen mittleren Durchmesser im Bereich von 100 bis 600 $\mu$m aufweisen und aus einem Kern, der mindestens den Wirkstoff enthält und mit mindestens einer Überzugsschicht überzogen ist, gebildet sind,
   - der Kern aus einem Trägerteilchen, das mit einer mindestens den Wirkstoff umfassenden Schicht beschichtet ist, gebildet ist,
   - die Überzugsschicht aus einem Material gebildet ist, das aus mindestens:

      - 25 bis 70 Gew.-%, bezogen auf das Gesamtgewicht des Überzugs, mindestens eines wasserunlöslichen Polymers A,
      - 30 bis 75 Gew.-%, bezogen auf das Gesamtgewicht des Überzugs, mindestens eines unterhalb von pH 5 wasserunlöslichen und oberhalb von pH 7 wasserlöslichen Polymers B und
      - 0 bis 25 Gew.-%, bezogen auf das Gesamtgewicht des Überzugs, mindestens eines Weichmachers besteht, wobei die Polymere A und B in einem Gewichtsverhältnis von Polymer(en) B/Polymer(en) A zwischen 0,75 und 4 vorliegen, und
      - die Überzugsschicht mindestens 35 Gew.-%, bezogen auf das Gesamtgewicht des Mikroteilchens, ausmacht,
      - die Wirkstoff-Mikroteilchen ein in-vitro-Freisetzungsprofil aufweisen, das **gekennzeichnet ist durch**:

         - eine Latenzzeit von weniger als 12 Stunden, speziell zwischen 0,5 und 8 Stunden oder sogar zwischen 1 und 5 Stunden mit anschließender verlängerter Freisetzung des Wirkstoffs in einem sauren wässrigen Medium mit einem pH-Wert von weniger als 4,
         - eine Beschleunigung der Freisetzung des Wirkstoffs nach Erhöhung des pH-Werts auf einen pH-Wert von mehr als 7.

2. Feste Form nach Anspruch 1, wobei die Überzugsschicht der Wirkstoff-Mikroteilchen weniger als 30 Gew.-% Talk, bezogen auf das Gesamtgewicht der Überzugsschicht, vorzugsweise weniger als 20 Gew.-%, insbesondere weniger als 10 Gew.-% und speziell weniger als 5 Gew.-% umfasst oder sogar frei von Talk ist.

3. Feste Form nach einem der vorhergehenden Ansprüche, wobei der Überzug der Mikroteilchen aus einer einzigen Schicht aus dem Material besteht.

4. Feste Form nach einem der vorhergehenden Ansprüche, wobei der auf der Oberfläche der Mikroteilchen angeordnete Überzug in einem Überzugsgehalt im Bereich von 35 bis 60 Gew.-%, speziell 40 bis 55 Gew.-% und spezieller 45 bis 55 Gew.-%, bezogen auf das Gesamtgewicht des Mikroteilchens, vorliegt.

5. Feste Form nach einem der vorhergehenden Ansprüche, wobei das Polymer A aus Ethylcellulose, Celluloseacetatbutyrat, Celluloseacetat, Ammonio-(meth)acrylat-Copolymeren, Poly(meth)acrylsäureestern und Mischungen davon ausgewählt ist.

6. Feste Form nach einem der vorhergehenden Ansprüche, wobei der Überzug der Mikroteilchen 30 bis 65 Gew.-%, speziell 35 bis 60 Gew.-%, speziell 35 bis 55 Gew.-% und speziell 35 bis 50 Gew.-% Polymer(e) A, bezogen auf sein Gesamtgewicht, enthält.

7. Feste Form nach einem der vorhergehenden Ansprüche, wobei das Polymer B aus Copolymer(en) von Methacrylsäure und Methylmethacrylat, Copolymer(en) von Methacrylsäure und Ethylacrylat, Celluloseacetatphthalat, Celluloseacetatsuccinat, Celluloseacetattrimellitat, Hydroxypropylmethylcellulosephthalat, Hydroxypropylmethylcelluloseacetatsuccinat, Carboxymethylcellulose, Schellack-Gummi, Polyvinylacetatphthalat und Mischungen davon ausgewählt ist.

8. Feste Form nach einem der vorhergehenden Ansprüche, wobei das Polymer B aus Copolymer(en) von Methacrylsäure und Methylmethacrylat, Copolymer(en) von Methacrylsäure und Ethylacrylat und Mischungen davon ausgewählt ist.

9. Feste Form nach einem der vorhergehenden Ansprüche, wobei der Überzug der Mikroteilchen 30 bis 70 Gew.-%, speziell 35 bis 65 Gew.-% und insbesondere 35 bis 60 Gew.-% Polymer(e) B, bezogen auf sein Gesamtgewicht, enthält.

10. Feste Form nach einem der vorhergehenden Ansprüche, wobei der Überzug der Mikroteilchen aus mindestens einer Mischung gebildet ist, die als Polymer A mindestens Ethylcellulose oder Celluloseacetatbutyrat oder Ammonio(meth)acrylat-Copolymer oder eine Mischung davon mit mindestens einem Copolymeren von Methacrylsäure und Ethylacrylat oder einem Copolymeren von Methacrylsäure und Methylmethacrylat oder einer Mischung davon als Polymer B umfasst.

11. Feste Form nach einem der vorhergehenden Ansprüche, wobei der Überzug die Polymere A und B in einem Gewichtsverhältnis von Polymer(en) B/Polymer(en) A zwischen 0,75 und 2 und spezieller zwischen 0,75 und 1,5 umfasst.

12. Feste Form nach einem der vorhergehenden Ansprüche, wobei das Trägerteilchen einen mittleren Durchmesser kleiner gleich 300 $\mu$m, vorzugsweise zwischen 50 und 250 $\mu$m und spezieller zwischen 70 und 150 $\mu$m aufweist.

13. Feste Form nach einem der vorhergehenden Ansprüche, wobei die überzogenen Mikroteilchen einen mittleren Durchmesser im Bereich von 150 bis 350 $\mu$m, spezieller 200 bis 300 $\mu$m und speziell 250 bis 300 $\mu$m besitzen.

14. Feste Form nach einem der vorhergehenden Ansprüche, **dadurch gekennzeichnet, dass** das viskositätserhöhende Mittel aus:

        - Polyacrylsäuren, insbesondere Carbomeren,
        - Polyalkylenglykolen, beispielsweise Polyethylenglykolen,
        - Polyalkylenoxiden, beispielsweise Polyethylenoxiden oder Polyoxyethylenen,
        - Polyvinylpyrrolidonen,
        - Gelatinen,
        - Polysacchariden, vorzugsweise ausgewählt aus Natriumalginat, Pektinen, Guar-Gummi, Xanthanen, Carrageenanen, Gellanen, Hydroxypropylcellulose, Hydroxypropylmethylcellulose, Methylcellulose, Hydroxyethylcellulose und Carboxymethylcellulose,
        - und Mischungen davon, ausgewählt ist, es sich speziell bei dem viskositätserhöhenden Mittel um ein Polyoxyethylen handelt, das insbesondere ein hohes Molekulargewicht besitzt und spezieller ein mittleres Molekulargewicht im Bereich von 1 Million g/mol bis ungefähr 8 Millionen g/mol aufweist.

15. Feste Form nach einem der vorhergehenden Ansprüche, wobei der Wirkstoff aus Amphetaminen, Analgetika, Apetitzüglern, Antidepressiva, Antiepileptika, Antiparkinsonmitteln, Anxiolytika, Barbituraten, Benzodiazepinen, Hypnotika, Narkotika, insbesondere Opioiden, Neuroleptika, Psychostimulantien und Psychotropika ausgewählt ist, insbesondere ein Narkotikum ist, spezieller aus Oxycodon, Oxymorphon, Hydromorphon, Hydrocodon, Tramadol und pharmazeutisch unbedenklichen Salzen davon ausgewählt ist.

16. Feste Form nach einem der vorhergehenden Ansprüche, **dadurch gekennzeichnet, dass** sie in Form einer Tablette oder einer Gelatinekapsel vorliegt.

17. Feste Form nach einem der vorhergehenden Ansprüche, **dadurch gekennzeichnet, dass** sie mindestens ein Sequestriermittel in Form von Mikroteilchen, die von den Wirkstoff-Mikroteilchen verschieden sind, umfasst, wobei das Sequestriermittel spezieller ausgewählt ist aus:

        - Natriumdodecylsulfat oder Natriumdocusat;
        - quaternären Ammoniumsalzen, speziell Trimethyltetradecylammoniumbromid oder Benzethoniumchlorid;
        - stark sauren Kationenaustauscherharzen, wenn der Wirkstoff in Lösung kationisch ist, oder stark alkalischen Anionenaustauscherharzen, wenn der Wirkstoff in Lösung anionisch ist, je nach der Polarität des Wirkstoffs, und Mischungen davon, und speziell, wenn der Wirkstoff in Lösung in kationischer Form vorliegt, aus:
- stark sauren Kationenaustauscherharzen wie sulfonierten Copolymeren von Styrol und Divinylbenzol und
- schwach sauren Kationenaustauscherharzen, wie vernetzten Copolymeren von Methacrylsäure und Divinylbenzol, oder Mischungen davon.

**Claims**

1. Solid oral pharmaceutical form with modified release of at least one active ingredient, containing at least microparticles containing said active ingredient and at least one viscosifying agent in a form isolated from said microparticles of active ingredient, **characterized in that**

   - said microparticles of active ingredient possess an average diameter ranging from 100 to 600 $\mu$m, and are formed by a core containing at least said active ingredient and coated with at least one coating layer,
   - said core is formed by a support particle covered by a layer comprising at least said active ingredient,
   - said coating layer is formed by a material composed of at least:

     - 25 to 70 % by weight relative to the total weight of said coating, of at least one water-insoluble polymer A which,
     - 30 to 75 % by weight relative to the total weight of said coating, of at least one polymer B which is insoluble in water below pH 5 and soluble in water above pH 7, and
     - 0 to 25 % by weight relative to the total weight of said coating, of at least one plasticizer,

   - said polymers A and B being in a polymer(s) B/ polymer(s) A weight ratio comprised between 0.75 and 4, and
   - said coating layer represents at least 35 % by weight, relative to the total weight of said microparticle
   - said microparticles of active ingredient have an in vitro release profile **characterized by**:

     - in an aqueous acid medium with a pH less than 4, a latency period of less than 12 hours, in particular between 0.5 and 8 hours, or even between 1 and 5 hours, followed by the sustained release of the active ingredient,
     - after increasing pH to a pH greater than 7, an acceleration of the release of the active ingredient.

2. Solid form according to claim 1, wherein the coating layer of said active ingredient microparticles contains less than 30 % by weight of talc, relative to the total weight of said coating layer, preferably less than 20 % by weight, in particular less than 10 % by weight, in particular less than 5 % by weight, and even is totally free of talc.

3. Solid form according to any one of the previous claims, wherein the coating layer of the microparticles is composed of a single layer formed by said material.

4. Solid form according to any one of the previous claims, wherein the coating arranged on the surface of the microparticles is present with a coating rate ranging from 35 to 60 % by weight, in particular 40 to 55 %, more particularly 45 to 55 % by weight relative to the total weight of said microparticle.

5. Solid form according to any one of the previous claims, wherein the polymer A is chosen from ethylcellulose, cellulose acetate butyrate, cellulose acetate, ammonio (meth)acrylate copolymers, poly(meth)acrylic acid esters, and mixtures thereof.

6. Solid form according to any one of the previous claims, wherein the coating of the microparticles contains 30 to 65 % by weight, in particular 35 % to 60 % by weight, in particular 35 % to 55 % by weight, in particular 35 to 50 % by weight of polymer(s) A relative to its total weight.

7. Solid form according to any one of the previous claims, wherein the polymer B is chosen from methacrylic acid and methyl methacrylate copolymer(s), methacrylic acid and ethyl acrylate copolymer(s), cellulose acetate phthalate, cellulose acetate succinate, cellulose acetate trimellitate, hydroxypropylmethylcellulose phthalate, hydroxypropylmethylcellulose acetate succinate, carboxymethylcellulose, shellac gum, polyvinyl acetate phthalate, and mixtures thereof.

8. Solid form according to any one of the previous claims, wherein the polymer B is chosen from the methacrylic acid and methyl methacrylate copolymer(s), the methacrylic acid and ethyl acrylate copolymer(s) and mixtures thereof.

9. Solid form according to any one of the previous claims, wherein the coating of the microparticles contains 30 to 70 % by weight, in particular 35 to 65 % by weight, in particular 35 % to 60 % by weight of polymer(s) B relative to its total weight.

10. Solid form according to any one of the previous claims, wherein the coating of the microparticles is formed by at least one mixture comprising, as polymer A, at least ethylcellulose or cellulose acetate butyrate or ammonio (meth)acrylate copolymer or a mixture thereof, with, as polymer B, at least one methacrylic acid and ethyl acrylate copolymer or a methacrylic acid and methyl methacrylate copolymer or a mixture thereof.

11. Solid form according to any one of the previous claims, wherein the coating comprises the polymers A and B in a polymer(s) B/polymer(s) A weight ratio comprised between 0.75 and 2, and more particularly between 0.75 and 1.5.

12. Solid form according to any one of the previous claims, wherein said support particle possesses an average diameter less than or equal to 300 $\mu$m, preferably comprised between 50 and 250 $\mu$m, more particularly between 70 and 150 $\mu$m.

13. Solid form according to any one of the previous claims, wherein said coated microparticles possess an average diameter ranging from 150 to 350 $\mu$m, more particularly from 200 to 300 $\mu$m, in particular from 250 to 300 $\mu$m.

14. Solid form according to any one of the previous claims, wherein the viscosifying agent is chosen from:

   - the polyacrylic acids, in particular the carbomers,
   - the polyalkylene glycols, for example the polyethylene glycols,
   - the polyalkylene oxides, for example the polyethylene oxides or polyoxyethylenes,
   - the polyvinylpyrrolidones,
   - the gelatins,
   - the polysaccharides, preferably chosen from sodium alginate, pectins, guar gum, xanthans, carrageenans, gellans, hydroxypropylcellulose, hydroxypropylmethylcellulose, methylcellulose, hydroxyethylcellulose and carboxymethylcellulose,
   - and mixtures thereof,

   in particular the viscosifying agent is a polyoxyethylene, in particular possessing a high molecular weight, and more particularly having an average molecular weight ranging from 1 million g/mole to approximately 8 million g/mole.

15. Solid form according to any one of the previous claims, wherein the active ingredient is chosen from the amphetamines, analgesics, anorexigens, antidepressants, antiepileptics, antiparkinsonians, anxiolytics, barbiturates, benzodiazepines, hypnotics, narcotics in particular the opioids, the neuroleptics, psychostimulants and psychotropics, in particular is a narcotic, more particularly chosen from oxycodone, oxymorphone, hydromorphone, hydrocodone, tramadol and their pharmaceutically acceptable salts.

16. Solid form according to any one of the previous claims, wherein it is presented in the form of a tablet or gelatin capsule.

17. Solid form according to any one of the previous claims, wherein it comprises at least one sequestering agent in the form of microparticles distinct from microparticles of active ingredient, said sequestering agent being chosen from:

   - sodium dodecyl sulphate or sodium docusate;
   - quaternary ammonium salts, in particular tetradecyl trimethyl ammonium bromide or benzethonium chloride;
   - strongly acid cation exchange resins when the active ingredient in solution is cationic, or strongly basic anion exchange resins when the active ingredient in solution is anionic, according to the polarity of the active ingredient, and mixtures thereof,

   and in particular, when the active ingredient in solution is in cationic form, from:

   - strongly acid cation exchange resins, such as the sulfonated copolymers of styrene and divinylbenzene, and
   - weakly acid cation exchange resins, such as the cross-linked copolymers of methacrylic acid and divinylbenzene or their salts.

**FIGURE 1**

**FIGURE 2**

**FIGURE 3**

**FIGURE 4**

5a

5b

**FIGURE 5**

**FIGURE 6**

**FIGURE 7**

**FIGURE 8**

**FIGURE 9**

**FIGURE 10**

**FIGURE 11**

**FIGURE 12**

**FIGURE 13**

**FIGURE 14**

**FIGURE 15**

**FIGURE 16**

**FIGURE 17**

**FIGURE 18**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2007054378 A

**Littérature non-brevet citée dans la description**

- Pharmacopée Européenne
- Pharmacopée